# EUROPEAN PATENT APPLICATION

(11) **EP 3 257 863 A1**
(43) Date of publication of application: **20.12.2017**
(21) Application number: 16305733.4
(22) Date of filing: 16.06.2016
(51) Int. Cl.: C07K 14/435

(54) **FLOUROUS METABOLICALLY STABLE PEPTIDE ANALOGS**

(71) Applicant: Université de Strasbourg, 67000 Strasbourg (FR); INSERM - Institut National de la Santé et de la Recherche Médicale, 75654 Paris Cedex 13 (FR); Centre National de la Recherche Scientifique CNRS, 75794 Paris Cedex 16 (FR)
(72) Inventor: BONNET, Dominique, 67118 Geispolsheim (FR); LLORENS CORTES, Catherine, 91440 Bures-sur-Yvette (FR); ITURRIOZ, Xavier, 91290 La Norville (FR)
(74) Representative: Novagraaf Technologies

(57) **Abstract**

The present invention relates to metabolically stable and non-immunogen analogs completely hydrosoluble at physiological pH, and their use for the prevention or treatment of diseases mediated by G-protein coupled receptor (GPCR), in particular (Central Nervous System) CNS and cardiovascular diseases or disorders.

## Description

### Field of the invention

The invention relates to metabolically stable and non-immunogen analogs completely hydrosoluble, and their use for the prevention or treatment of diseases mediated by G-protein coupled receptor (GPCR), in particular (Central Nervous System) CNS and cardiovascular diseases or disorders.

### Background of the invention

To date more than 7000 naturally occurring peptides have been identified, and these often have a crucial role in human physiology including actions as hormones, neurotransmitters, growth factors, ion channel ligands, or anti-infectives. In general, peptides are selective and efficacious signaling molecules that bind to specific cell surface receptors, such as G-protein coupled receptors (GPCRs) or ion channels, where they trigger intracellular effects. Thereby, during the last decade, peptides have gained a wide range of applications in medicine. Indeed, more than 60 US Food and drug Administration (FDA)-approved peptide medicines are on the market and this is expected to grow significantly, with approximately 140 peptide drugs currently in clinical trials and more than 500 therapeutic peptides in preclinical development (Fosgerau, K.; Hoffmann, T. Peptide therapeutics: current status and future directions. Drug Discovery Today 2015, 20, 122-128) [1].

However, naturally occurring peptides are often not directly suitable for use as convenient therapeutics because they have intrinsic weaknesses, including poor chemical and physical stability, and a short circulating plasma half-life. (Vlieghe, P.; Lisowski, V.; Martinez, J.; Khrestchatisky, M. Synthetic therapeutic peptides: science and market. Drug Discovery Today 2010, 15, 40-56) [2].

The techniques developed for half-life extension are generally based on the modification of the native peptide backbone (non-natural aminoacids, lactam bridges, stapling, cyclization). Polyethylene glycol (PEG) incorporation into peptides has also been used to limit glomerular filtration and thereby increasing plasma half-life by limiting the elimination of peptides. However, because of increased safety and tolerability concerns relating to the use of PEG as a component of an injectable therapeutic, PEGylation has become a less preferred choice. Another approach is based on the binding of the peptide to the circulating protein albumin used as a vehicle by peptide acylation with hydrocarbon tail as seen in the GLP-1 agonist. Nevertheless the presence of the hydrocarbon tail makes the peptide less selective for its target with potential enhanced toxicity (Fosgerau, K.; Hoffmann, T. Peptide therapeutics: current status and future directions. Drug Discovery Today 2015, 20, 122-128) [1].

G-protein coupled receptors (GPCRs), also known as seven-transmembrane domain receptors, 7TM receptors, heptahelical receptors, serpentine receptors, and G protein-linked receptors (GPLR), constitute a large family of receptors, the G-protein-coupled receptor (GPCR) family (a superfamily/family within the transporter-opsin-G (TOG) protein-coupled receptors superfamily), that sense molecules outside the cell and activate inside signal transduction pathways and ultimately, cellular responses. Coupling with G proteins, they are called seven-transmembrane receptors because they pass through the cell membrane seven times. G protein-coupled receptors are found only in eukaryotes, including yeast, choanoflagellates, and animals. The ligands that bind and activate these receptors include light-sensitive compounds, odors, pheromones, hormones, and neurotransmitters, and vary in size from small molecules to peptides to large proteins. G protein-coupled receptors are involved in many diseases, and are also the target of approximately 40% of all modern medicinal drugs (Congreve, M.; Langmead, C. J.; Mason, J. S.; Marshall, F. H. Progress in Structure Based Drug Design for G Protein-Coupled Receptors. J. Med. Chem. 2011, 54, 4283-4311) [3].

As an example of GPCRs peptide, apelin serves important function in food intake, vasopressin and histamine release, gastric acid, bicarbonate secretion and insulin secretion, diuresis, cell proliferation, angiogenesis, glucose-fluid balance and regulation of gastrointestinal motility and cardiovascular system. (Narayanan S, Harris DL, Maitra R, Runyon SP. Regulation of the Apelinergic System and Its Potential in Cardiovascular Disease: Peptides and Small Molecules as Tools for Discovery. J Med Chem. 2015 Oct 22;58(20):7913-27) [4].

As another example of GPCRs peptide, angiotensin behaves as an antagonist of AT1R. It is well established that the clinical benefits of AT1R blockers extend far beyond their control of blood pressure, and include the reduction of mortality from myocardial infarction, heart failure, atherosclerosis, stroke, diabetes, peripheral vascular disease and chronic renal disease (Marc Y, Llorens-Cortes C. The role of the brain renin-angiotensin system in hypertension: implications for new treatment.. Prog Neurobiol. 2011 Oct;95(2):89-103) [5].

As another example of GPCRs peptide, there is growing evidence that the neuropeptide oxytocin (OT) modulates complex social behavior and social cognition. OT has been reported in human to improve prosocial behavior and trust, social memory, to decrease fear associated with social phobia (Modi, M. E.; Young, L. J. The oxytocin system in drug discovery for autism: Animal models and novel therapeutic strategies. Horm. Behav. 2012, 61, 340-350) [6].

However insofar as the half-life of apelin, angiotensin or oxytocin in the blood circulation is short, there is a need of designing, synthesizing and testing novel potent and more stable peptides derived therefrom that can activate the GPCR pathways.

### Summary of the invention

The invention relates to new fluorocarbon conjugates to increase therapeutic peptides half-life, in particular GPCR peptides half-life, by means of supramolecular polymerization, thus leading to fluoropeptides metabolically more stable, non-immunogen and completely hydrosoluble at physiological pH. Such compounds constitute potential new therapeutic agents to prevent or treat diseases mediated by the GPCRs without increasing humoral and/or cellular immunogenicity to them.

In one aspect, the present invention relates to a metabolically stable and non-immunogenic peptide analog completely hydrosoluble at physiological pH having a peptide covalently linked to a fluorocarbon group, directly or through a linker selected from the group consisting of a PEG or a peptide having from 1 to 6 amino acids, either on the alpha-amino or the epsilon-amino group of at least one lysine of said peptide, when the linker is a lysine, the fluorocarbon group is directly linked to the epsilon-amino group of said linker;
but excluding an apelin analog having the following peptide of formula (I):

Lys-Phe-Xaa1-Arg-Xaa2-Arg-Pro-Arg-Xaa3-Ser-Xaa4-Lys-Xaa5-Pro-Xaa6-Pro-Xaa7 (I)

wherein said peptide of formula (I) (SEQ ID NO: 1) is linked to a fluorocarbon group, an acetyl group, or an acyl group -C(O)R where R is a C₇₋₃₀ alkyl, directly or through a linker selected from the group consisting of PEG, lysine and arginine, either on the alpha-amino or the epsilon-amino group of at least one lysine of the peptide formula (I), and when the linker is a lysine, the fluorocarbon group, acetyl or acyl group is directly linked either on the epsilon-amino group of said linker and wherein :
Xaa1 is arginine (R) or D-isomer arginine (R_{D});
Xaa2 is glutamine (Q) or D-isomer glutamine (Q_{D})
Xaa3 is leucine (L) or D-isomer leucine (L_{D});
Xaa4 is histidine (H) or α-aminoisobutyric acid (Aib);
Xaa5 is alanine (A) or D-isomer alanine (A_{D}) or glycine (G);
Xaa6 is methionine (M) or norleucine (Nle);
Xaa7 is phenylalanine (F) of 4-Br phenylalanine (4-BrF).

According to a particular embodiment of the present invention, the peptide analog of the present invention has a peptide covalently linked to the fluorocarbon group that is no more than 13 amino acid residues.

As used herein, the expression "completely hydrosoluble at physiological pH" means that the fluoropeptides of the present invention as above described have at least 50% hydrophobic amino acid residues and have an overall positive net charges and a final solubility in aqueous mixture above 100 µM by visual inspection of the cloudiness of the resulting dispersion and/or solubility measurements by classical physicochemical methods.

As used herein, the expression "non-immunogen" means that the fluoropeptides of the present invention as above described are not derived from any antigens capable of inducing immune response in an animal, including humans. Antigens may be derived from a virus, bacterium or mycobacterium, parasite, fungus, or any infectious agent or an autologous antigen or allergen. The fluoropeptides described in the invention are not included in any vaccine.

As used herein, the expression "metabolically stable" means that the fluoropeptides of the present invention as above described, have at least the half-life of the native peptide, in particular have a half-life at twice longer than native peptide, or at least 20 min or more than one hour, as measured in the stability assay performed in human plasma.

As used herein, the expression "fluorocarbon group" includes either, perfluorocarbon (where all hydrogen are replaced by fluor) or, hydrofluorocarbon (which contains both C-H and C-F bonds).

The fluorocarbon group may comprise one or more chains derived from perfluorocarbon or mixed fluorocarbon/hydrocarbon radicals, and may be saturated or unsaturated, each chain having from 3 to 30 carbon atoms. The fluorocarbon group is linked to the peptide through a covalent linkage, for example via NH₂-group of a lysine of the peptide of formula (I). The coupling to the peptide may be achieved through a functional group for linkage to -NH₂, being naturally present on the lysine of the peptide of formula I, or onto a linker. Modify the nature of the linkage between the fluorocarbon chain and the peptide allows to modulate the stability and/or solubility of the peptide. Examples of such linkages between the fluorocarbon chain and the peptide of formula I include amide, hydrazine, disulphide, thioether, ester and oxime bonds.

Optionally, a cleavable linker element (peptide or non-peptidic) may be incorporated to permit cleavage of the peptide from the fluorocarbon group. The linker may also be incorporated to assist in the synthesis of the fluoropeptide and to improve its stability and/or solubility, for example by including additional charges. So charged linker may be particularly useful especially if the peptide to which it is linked has no cationic aminoacids (*i.e.* lysine, histidine, arginine) at its N-terminal end. Examples of linkers include polyethylene glycol (PEG), or a peptide having about 1 to 6 amino acids, natural on non-natural ones, that may be cleaved by proteolytic enzymes or not. Preferably said amino acids are chosen from the group consisting of basic or aliphatic amino acids, more preferably from histidine, lysine, arginine and glycine. For example, the linker may be Arg-Gly-Arg.

Thus, the fluorocarbon group of the peptide analog of the present invention has chemical formula (II) CₘFₙ-C_{y}Hₓ(L) wherein m=3 to 30, n≤2m+1, y=0 to 2, x≤2y, (m+y)=3 to 30, and L, which is optional, is a functional group leading to covalent attachment to the peptide. For example said functional group is a carbonyle -C(O)- that forms an amide bond with the -NH2 of a lysine.

According to a particular embodiment of the above formula II of the fluorocarbon, m=5 to 15, preferably m=5 to 15 and y=1 to 4. According to another particular embodiment, the formula of the fluorocarbon group is perfluoroundecanoic acid of formula (A) 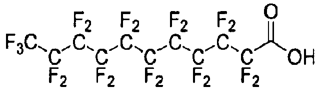 or alternatively is 2H,2H,2H,3H,3H-perfluoroundecanoic acid of formula (B) 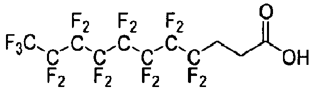 or alternatively is heptadecafluoro-pentadecanoic acid of formula (C)

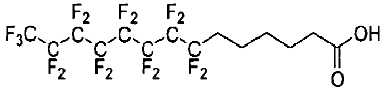

In these cases it is to be noted that reducing the length of the fluorocarbon group of formula (II), for example by deleting at least two CF₂ groups, preferably at least four CF₂ groups, can increase the solubility and/or plasmatic stability of the peptide to which it is linked.

According to a particular embodiment, the peptide analog of the present invention as above defined is further covalently linked to an acetyl group and/or an acyl group -C(O)R where R is a C₇₋₃₀ alkyl. For example it has the following formula (III) CH₃-C_{y}Hx-C(O)- wherein y=7 to 30, preferably y=10 to 20, more preferably y=14, and x=2y.

Said fluorocarbon group or further acetyl and/or acyl group can be linked at the N-terminal part of the peptide directly through a lysine, either on the alpha-amino or the epsilon-amino groups.

In one aspect, the peptide analog of the present invention includes but are not limited to neuropeptides or peptide hormones, a peptide mimetics or a fragment derived therefrom, for example Adiponectin, Adrenomedullin, Angiotensinogen, Angiotensin I, Angiotensin II, , Angiopoietin-like Protein 8/Betatrophin, Amylin, Apelin, Apela Asprosin, Atrial Natriuretic Peptide/ANP0 BNP, Betatrophin, Bradykinin, Calcitonin , Cholecystokinin, Chorionic Gonadotropin alpha Chain (HCG alpha), Chorionic Gonadotropin alpha/beta (HCG), CILP-1, Claudin-4, CNP, Copeptin, CRHBP, Corticotropin-releasing hormone, Dopamine, Enkephalin, Endothelin, Endothelin-1, Endothelin-2, Endothelin-3, Erythropoietin, FSH alpha/beta, FSH beta, , Gastrin I, Gastrin-releasing Peptide/Bombesin, Gastric inhibitory polypeptide (GIP), Ghrelin/Obestatin, Growth hormone-releasing hormone (GHRH)Glucagon, GLP1, GLP2, Gonadotropin-releasing hormone (GnRH)GOAT/MBOAT4, Growth Hormone, Growth Hormone 2, Hepcidin, IGF-I, IGF-II, IGFBP-1, IGFBP-2, IGFBP-3, IGFBP-4, IGFBP-5, IGFBP-6, IGFBP-L1, IGFBP-rp1/IGFBP-7, IGFL-3, Incretins, INSL3, INSL4, INSL6, Insulin, Parathyroid hormone (PTH), Proinsulin, Irisin/FNDC5, Leptin/OB, LH alpha/beta Heterodimer, LH beta, Mas, , MCT8/SLC16A2, , Melanocyte-stimulating hormone, melatonin, Metastin/KiSS1, Motilin, Neuropeptide S/NPS, Neuropeptide Y/NPY, Neurophysin II, Obestatin, Orexin A/Hypocretin-1, Orexin B/Hypocretin-2, Osteocalcin, Oxytocin, PACAP/ADCYAP1, Pancreatic Polypeptide/PP, PITX2, Peptide YY (PYY), Placental Lactogen/CSH1, Prolactin, Proliferin, Proliferin-related Protein/Plfr, Proprotein Convertase 2/PCSK2, PSG-1, PSG-6, PTH, PTHLH/PTHrP, Retinol Binding Protein 4, Relaxin-1, Relaxin-2, Relaxin-3, Renalase, Secretin, Serpin A8/Angiotensinogen, SOCS-2, Somatostatin, , Stanniocalcin 1/STC-1, Stanniocalcin 2/STC-2, Substance P, Thrombopoietin, Thyroglobulin, Thyroxine, Transthyretin/Prealbumin Thyrotropin-releasing hormone (TRH), TSH alpha/beta Heterodimer, TSH beta, Urocortin, Urotensin-II, V1 b Vasopressin/Arg8-Vasopressin, VIP.

In one aspect, the peptide analog of the present invention includes but are not limited to natural or non-natural peptide which is capable of interacting with AGTR-1, AGTR-2, Bradykinin RB1/BDKRB1, BRS3, C5L2/GPR77, Calcitonin R, CCK-A R, Cholecystokinin-B R/CCKBR, Pro-Corticoliberin/Pro-CRH, CRHR1, CRHR2, CRLR, EDNRA/Endothelin R Type A, ELTD1, Endothelin, EDNRB/Endothelin R Type B, Erythropoietin R, FSH R, Gastrin-releasing Peptide R/GRPR, GHRHR, GHSR, GIPR, GLP-1R, GLP-2R, Glucagon R/GCGR, GnRHR, GPR10, GPR39, Growth Hormone R/GHR, IGF-I R, IGFLR1, Insulin R/CD220, Insulin R/IGF-I R Heterotetramer, INSRR, IRS1, IRS2, KiSS1R/GPR54, Leptin R, Lgr6, LHR, MCHR1, MCHR2 Melanocortin-1 R/MC1R, Melanocortin-2 R/MC2R, Melanocortin-3 R/MC3R, Melanocortin-4 R, Melanocortin-5 R/MC5R, Motilin R/GPR38, NK1R, NK2R, NK3R/TACR3, NPR-1, Orexin R1/HCRTR1, Orexin R2/HCRTR2, OXTR, Prokineticin R1/PROKR1, Prokineticin R2/PROKR2, Prolactin R, PTH1R/PTHR1, PTH2R, RAMP1, RAMP2, RAMP3, Relaxin R1, Relaxin R2, RXFP3/RLN3R1, RXFP4/GPCR142, Secretin R, Somatostatin R1/SSTR1, Somatostatin R3/SSTR3, Somatostatin R4/SSTR4, Somatostatin R2/SSTR2, Somatostatin R5/SSTR5, TRHR, TSH R, Urotensin-II R, Vasopressin R/AVPR1B, V2 Vasopressin R/AVPR2, V1a Vasopressin R/AVPR1A, VIP R1/VPAC1

According to a particular embodiment, the peptide analog of the present invention is selected from:
i) an apelin analog having said peptide of the formula (IV) (SEQ ID NO: 2):

   Xaa1-Arg-Pro-Xaa2-Leu-Xaa3-Xaa4-Xaa5-Gly-Pro-Xaa6-Pro-Xaa7 (IV)

   and wherein
   Xaa1 is L- or D-glutamine (QL or QD) or alanine (A);
   Xaa2 is arginine (R) or lysine (K) or D-norleucine (Nle);
   Xaa3 is serine (S) or alanine (A);
   Xaa4 is histidine (H) or alanine (A);
   Xaa5 is lysine (K) or norleucine (Nle);
   Xaa6 is methionine (M), leucine (L), phenylalanine (F) or norleucine (Nle); Xaa7 is phenylalanine (F), 4-Br phenylalanine (4-BrF), 4-(Obenzyl)phenylalanine (4-ObnF) or p-benzoyl phenylalanine (Bpa); and
ii) an apelin analog with an amino acid sequence having at least 80% identity with the sequence of (i).

According to another particular embodiment, a peptide analog of the present invention is selected from the group consisting of:
i) an angiotensin II analog having said peptide of the formula (V) (SEQ ID NO: 3):

   Xaa1-Arg-Val-Tyr-Ile-His-Pro-Xaa2 (V)

   and wherein
   Xaa1 is aspartic acid (D) or sarcosine;
   Xaa2 is phenylalanine or OH; and
ii) an angiotensin II analog with an amino acid sequence having at least 80% identity with the sequence of (i).

According to another particular embodiment, a peptide analog of the present invention is selected from the group consisting of:
i) an oxytocin analog having said peptide of the formula (VI) (SEQ ID NO: 4):

   Cys- Tyr-Ile-Xaa1-Asp-Cys-Xaa2-Xaa3-Gly (VI)

   and wherein
   Xaa1 is glutamine or threonine;
   Xaa2 is proline or glycine;
   Xaa3 is leucine, lysine or proline;
ii) an oxytocin analog with an amino acid sequence having at least 80% identity with the sequence of (i).

In another aspect, the present invention also relates to a peptide analog of the present invention, for use as a drug.

In another aspect, the present invention also relates to a peptide analog of the present invention, for use in the prevention and/or treatment of a GPCR-related disease or GPCR-related disorder, preferably selected from central nervous system (CNS) disorders, cardiovascular disorders, nociception, renal disorders, neuroinflammation, etc.... For example said diseases or disorders include, but are not limited to:
- cardiovascular disease: heart failure, kidney diseases (*e.g*. renal failure, nephritis, etc...), hypertension, pulmonary hypertension, cirrhosis, arteriosclerosis, pulmonary emphysema, pulmonary oedema, stroke, brain ischemia, myocardial impairment in sepsis, cardiomyopathy;
- the syndrome of inappropriate antidiuretic hormone (SIADH);
- metabolic diseases: obesity, anorexia, hyperphagia, polyphagia, hypercholesterolemia, hyperglyceridemia, hyperlipemia;
- various types of dementia: senile dementia, Alzheimer's disease, cerebrovascular dementia, dementia due to genealogical denaturation degenerative disesases, dementia resulting from infectious diseases, dementia associated with endocrine diseases, metabolic diseases, or poisoning, dementia caused by tumors, and dementia due to traumatic diseases, depression, hyperactive child syndrome, disturbance of consciousness, anxiety disorder, schizophrenia, phobia;
- pain and hyperalgesia.

For example said therapeutic peptides of the present invention include, but are not limited to the following peptides :
In cardiovascular disease :

| | |
|---|---|
| Balixafortide | ACSAPRYCYQKPPYH (SEQ ID NO: 5) |
| BIS-5409 / MR-409 | YADAIFTNSYRKVLGQLSARKLLQDIMSR (SEQ ID NO: 6) |
| Bivalirudin | FPRPGGGGNGDFEEIPEEYL (SEQ ID NO: 7) |
| BQ-123 | DPVLY (SEQ ID NO: 8) |
| Carperitide | SLRRSSCFGGRMDRIGAQSGLGCNSFRY (SEQ ID NO: 9) |
| CEL-1000 | DGQEEKAGVVSTGLIGGG (SEQ ID NO: 10) |
| Cenderitide | GLSKGCFGLKLDRIGSMSGLGCPSLRDPRPNAPSTSA (SEQ ID NO:11) |
| CGEN-856S | FLGYCIYLNKRRGDPAFKRRLRD (SEQ ID NO: 12) |
| Des-Asp Angiotensin 1 | DRVYIHPFHL ((SEQ ID NO: 13) |
| Desirudin | VVYTDCTESGQNLCLCEGSNVCGQGNKCILGSDGEKNQCVTGE GTPKPQSHNDGDFEEIPEEYLQ (SEQ ID NO: 14) |
| Elamipretide | R-2,6-dimethyl-YKF (SEQ ID NO: 15) |
| FX-06 | RGHRPLDKKREEAPSLRPAPPPISGGGY (SEQ ID NO: 16) |
| LJPC-501 (Angiotensin II) | DRVYIHPF (SEQ ID NO: 17) |
| MP-3167 (Vastiras) | AVSNADLMDFKNLLDHLEEKMPLEDEVVPPQVLSERN (SEQ ID NO:18) |
| NA-1 | YRKKRRQRRRKLSSIESDV (SEQ ID NO: 19) |
| NBI-69734 (Urocortin II) | IVLSLDVPIGLLQILLEQARARAAREQATTNARILARVGH (SEQ ID NO: 20) |
| Nesiritide | SPKMVQGSGCFGRKMDRISSSSGLGCKVLRRH (SEQ ID NO: 21) |
| PL-3994 | CHFAGRXDRISCYR; X = Nle (SEQ ID NO: 22) |
| RGN-352 (Thymosin beta-4) | MSDKPDMAEIEKFDKSKLKKTETQEKNPLPSKETIEQEKQAGES (SEQ ID NO: 23) |
| Selepressin | (2S)-1-[(4R,7S,10S,13S,16S,19R)-19-amino-10-(4-amino-4-oxobutyl)-7-(2-amino-2-oxoethyl)-16-benzyl-13-[(2S)-butan-2-yl]-6,9,12,15,18-pentaoxo-1,2-dithia-5,8,11,14,17-pentazacycloicosane-4-carbonyl]-N-[(2S)-1-[(2-amino-2-oxoethyl)amino]-1-oxo-5-(propan-2-ylamino)pentan-2-yl]pyrrolidine-2-carboxamide |
| Solnatide | CGQRETPEGAEAKPWYC (SEQ ID NO: 24) |
| Terlipressin | (2S)-1-[(4R,7S,10S,13S,16S,19R)-19-[[2-[[2-[(2-aminoacetyl)amino]acetyl]amino]acetyl]amino]-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]-N-[(2S)-6-amino-1-[(2-amino-2-oxoethyl)amino]-1-oxohexan-2-yl]pyrrolidine-2-carboxamide |
| THR-18 | (4S)-4-[[(2S)-1-[(2S)-2-[[(2S)-2-[[(2S)-2-acetamido-5-carbamimidamidopentanoyl]amino]-4-methylsulfanylbutanoyl]amino]propanoyl]pyrrolidine-2-carbonyl]amino]-5-[[(2S)-1-[[(2S,3S)-1-[[(2S,3S)-1-[[(2S)-1-[[(2S)-1-[[(2S)-1-[(2S)-2-[[1-[[(2S)-1-[[(2S)-1-[[(2S)-1-[[1-[[(2S)-1-amino-5-carbamimidamido-1-oxopentan-2-yl]amino]-3-methyl-1-oxobutan-2-yl]amino]-3-methyl-1-oxobutan-2-yl]amino]-3-(4-hydroxyphenyl)-1-oxopropan-2-yl]amino]-4-methyl-1-oxopentan-2-yl]amino]-1-oxo-3-phenylpropan-2-yl]carbamoyl]pyrrolidin-1-yl]-5-carbamimidamido-1-oxopentan-2-yl]amino]-3-carboxy-1-oxopropan-2-yl]amino]-4-methylsulfanyl-1-oxobutan-2-yl]amino]-3-methyl-1-oxopentan-2-yl]amino]-3-methyl-1-oxopentan-2-yl]amino]-4-carboxy-1-oxobutan-2-yl]amino]-5-oxopentanoic acid;2,2,2-trifluoroacetic acid |
| TRV027 | GRVYIHPA (SEQ ID NO: 25) |
| Ularitide (Urodilatin) | TAPRSLRRSSCFGGRMDRIGAQSGLGCNSFRY (SEQ ID NO: 26) |

In various central nervous system diseases :

| | |
|---|---|
| acALY-18 | ALYDKGYTSKEQKDCVG (SEQ ID NO: 27) |
| Cibinetide | 5-Oxo-PEQLERALDSS (SEQ ID NO: 28) |
| COG-112 | RQIKIWFQNRRMKWKKC (SEQ ID NO: 29) |
| corticotropin | SYSMEHFRWGKPVGKKRRPVKVYPDGAEDQLAEAFPLEF (SEQ ID NO: 30) |
| CR-845 (Difelikefalin) | 4-Piperidinecarboxylic acid, 4-amino-1-[(2R)-6-amino-2-[[(2R)-2-[[(2R)-2-[[(2R)-2-amino-1-oxo-3-phenylpropyl]amino]-1-oxo-3-phenylpropyl]amino]-4-methyl-1-oxopentyl]amino]-1-oxohexyl] |
| davunetide | (2S)-5-amino-2-[[(2S)-1-[(2S,3S)-2-[[(2S)-2-[[(2S)-2-[[(2S)-1-[(2S)-2-[[(2S)-2,4-diamino-4-oxobutanoyl]amino]propanoyl]pyrrolidine-2-carbonyl]amino]-3-methylbutanoyl]amino]-3-hydroxypropanoyl]amino]-3-methylpentanoyl]pyrrolidine-2-carbonyl]amino]-5-oxopentanoic acid |
| elcatonin | 1,7-Dicarbacalcitonin (salmon), 1-butanoic acid-26-L-aspartic acid-27-L-valine-29-L-alanine- |
| glatiramer acetate | acetic acid;2-amino-3-(4-hydroxyphenyl)propanoic acid;2-aminopentanedioic acid;2-aminopropanoic acid;2,6-diaminohexanoic acid |
| leuprorelin ER | N-[1-[[1-[[1-[[1-[[1-[[1-[[5-(diaminomethylideneamino)-1-[2-(ethylcarbamoyl)pyrrolidin-1-yl]-1-oxopentan-2-yl]amino]-4-methyl-1-oxopentan-2-yl]amino]-4-methyl-1-oxopentan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide |
| PMZ-2123 | DPVLW (SEQ ID NO: 31) |
| PT-320 | SHGEGTFATSDLSKQMQQQAVRLFIQWLKDGGPSSGAPPP (SEQ ID NO: 32) |
| Relamorelin | 4-[[(2S)-2-[[(2R)-2-[[(2R)-3-(1-benzothiophen-3-yl)-2-(piperidine-3-carbonylamino)propanoyl]amino]-3-(1H-indol-3-yl)propanoyl]amino]-3-phenylpropanoyl]amino]piperidine-4-carboxamide |
| Ziconotide | CKGKGAKCSRLMYDCCTGSCRSGKC (SEQ ID NO: 33) |

In various Dermatologic diseases :

| | |
|---|---|
| AB-103 | SPMLVAYD (SEQ ID NO: 34) |
| AG-30/5C | MLKLIFLHRLKRMRKRLKRKLRLWHRKRYK (SEQ ID NO: 35) |
| Bacitracin | (4R)-5-[[1-[[(3R,9R,15R)-15-(3-aminopropyl)-9-benzyl-12-butan-2-yl-3-(carboxymethyl)-6-(1H-imidazol-5-ylmethyl)-2,5,8,11,14,17-hexaoxo-1,4,7,10,13,16-hexazacyclodocos-18-yl]amino]-3-methyl-1-oxopentan-2-yl]amino]-4-[[4-methyl-2-[[2-(3-m ethyl pentan-2-yl)-4,5-dihydro-1,3-thiazole-4-carbonyl]amino]pentanoyl]amino]-5-oxopentanoic acid |
| Desloratadine | 8-chloro-11-piperidin-4-ylidene-5,6-dihydrobenzo[1,2]cyclohepta[2,4-b]pyridine |
| FOL-005 | VDTYDGDISWYGLR (SEQ ID NO: 67) |
| Granexin | RQPKIWFPNRRKPWKKRPRPDDLEI (SEQ ID NO: 37) |
| LL-37 | LLGDFFRKSKEKIGKEFKRIVQRIKDFLRNLVPRTES (SEQ ID NO: 38) |
| Omiganan | ILRWPWWPWRRK (SEQ ID NO: 39) |
| PXL-01 | EATKCFQWQRNMRKVRGPPVSCIKR (SEQ ID NO: 40) |
| RGN-137 | MSDKPDMAEIEKFDKSKLKKTETQEKNPLPSKETIEQEKQAGES (SEQ ID NO: 41) |
| SR-0379 | MLKLIFLHRLKRMRKRLKRK (SEQ ID NO: 42) |

In various ear nose throat disorders :

| | |
|---|---|
| B-2088 | RGRKVVRRKKRRVVKRGR (SEQ ID NO: 43) |
| XG-102 | DQSRPVQPFLNLTTPRKPRPPRRRQRRKKRG (SEQ ID NO: 44) |

In various gastrointestinal diseases :

| | |
|---|---|
| Colistin Sulfate | N-[(2S)-4-amino-1-[[(2S,3R)-1-[[(2S)-4-amino-1-oxo-1-[[(3S,6S,9S, 12S, 15R, 18S,21S)-6,9,18-tris(2-aminoethyl)-3-[(1R)-1-hydroxyethyl]-12,15-bis(2-methylpropyl)-2,5,8,11,14,17,20-heptaoxo-1,4,7,10,13,16,19-heptazacyclotricos-21-yl]amino]butan-2-yl]amino]-3-hydroxy-1-oxobutan-2-yl]amino]-1-oxobutan-2-yl]-6-methyloctanamide;sulfuric acid |
| Cosyntropin ER | SYSMEHFRWGKPVGKKRRPVKVYP (SEQ ID NO: 45) |
| FE-203799 | HGDGSFSDEMFTILDLLAARDFFINWLIQTKITD (SEQ ID NO: 46) |
| Linaclotide | CCEYCCNPACTGCY (SEQ ID NO: 47) |
| NB-1001 | GLP-1-XTEN |
| Octreotide | FCFWKXCX X : DL-Threonine (SEQ ID NO: 48) |
| Octreotide Acetate | FCFWKTCT (SEQ ID NO: 49) |
| Plecanatide | NDECELCVNVACTGCL (SEQ ID NO: 50) |
| Recanaclotide | CCXLCCNPACTGC X : Ser(PO3H2) (SEQ ID NO: 51) |
| Somatostatin | AGCKNFFWKXFXSC X : DL-Threonine (SEQ ID NO: 52) |
| Teduglutide | HGDGSFSDEMNTILDNLAARDFINWLIQTKITD (SEQ ID NO: 53) |
| Vapreotide Acetate | acetic acid;10-(4-aminobutyl)-N-[1-amino-3-(1H-indol-2-yl)-1-oxopropan-2-yl]-19-[(2-amino-3-phenylpropanoyl)amino]-16-[(4-hydroxyphenyl)methyl]-13-(1H-indol-3-ylmethyl)-6,9,12,15,18-pentaoxo-7-propan-2-yl-1,2-dithia-5,8,11,14,17-pentazacycloicosane-4-carboxamide |
| Vasopressin | N-[6-amino-1-[(2-amino-2-oxoethyl)amino]-1-oxohexan-2-yl]-1-[19-amino-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]pyrrolidine-2-carboxamide; 1-[19-amino-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]-N-[1-[(2-amino-2-oxoethyl)amino]-5-(diaminomethylideneamino)-1-oxopentan-2-yl]pyrrolidine-2-carboxamide |
| ZP-1848 | HGEGTFSSELATILDALAARDFIAWLIATKITD (SEQ ID NO: 54) |

In various genetic disorders diseases :

| | |
|---|---|
| Afamelanotide | SYSXEHFRWGKPV ; X : Nle (SEQ ID NO: 55) |
| Alisporivir | (3S,6S,9S, 12R, 15S, 18S,21 S,24S,27R,30S,33S)-25,30-diethyl-33-[(E,1R,2R)-1-hydroxy-2-methylhex-4-enyl]-1,4,7,10,12,15,19,27,28-nonamethyl-6,9,18-tris(2-methylpropyl)-3,21,24-tri(propan-2-yl)-1,4,7,10,13,16,19,22,25,28,31-undecazacyclotritriacontane-2,5,8,11,14,17,20,23,26,29,32-undecone |
| AZP-531 | SPEHQRVQ (SEQ ID NO: 56) |
| LJPC-401 | DTHFPICIFCCGCCHRSKCGMCCKT (SEQ ID NO: 57) |
| Setmelanotide | RCAHFRWC (SEQ ID NO: 58) |
| TXA-127 | DRVYIHP (SEQ ID NO: 59) |

In various genito-urinary system and sexual hormones disorders :

| | |
|---|---|
| AMY-101 | YICVWQDWXHRCI; X : Sar (SEQ ID NO: 60) |
| Cardiotoxin | LKCNKLVPLFYKTCPAGKNLCYKMFMVSNKMVPVKRGCIDVCPKSS LLVKYVCCNTDRCN (SEQ ID NO: 61) |
| Desmopressin | (2S)-N-[(2R)-1-[(2-amino-2-oxoethyl)amino]-5-(diaminomethylideneamino)-1-oxopentan-2-yl]-1-13S,16S)-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]pyrrolidine-2-carboxamide |
| Lypressin | N-[6-amino-1-[(2-amino-2-oxoethyl)amino]-1-oxohexan-2-yl]-1-[19-amino-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]pyrrolidine-2-carboxamide |

In various hematologicaL disorders :

| | |
|---|---|
| Aprotinin | RPDFCLEPPYTGPCRARIIRYFYNAKAGLCQTFVVGGCRAKRNNFKS AEDCERTCGGA (SEQ ID NO: 62) |
| BL-8040 | RRX₁CYXKKPYRXCR ; X1:Nal, X: Cit (SEQ ID NO: 63) |
| CBLB-612 | VQGEESNDK (SEQ ID NO: 64) |
| EPO-018B | GGTYSCHFGALTWVCRPQRGA (SEQ ID NO: 65) |
| Peginesatide | Poly(oxy-1,2-ethanediyl), alpha-hydro-omega-methoxy-, diester with 21N6,21'N6-[[(N2,N6- dicarboxylysyl-beta-alanyl)imino]bis(1-oxo-2,1-ethanediyl)]bis[N-acetylglycylglycyl-L- leucyl-L-tyrosyl-L-alanyl-L-cysteinyl-L-histidyl-L-methionylglycyl-L-prolyl-L-isoleucyl-L- threonyl-3-(1-naphthalenyl)-L-alanyl-L-valyl-L-cysteinyl-L-glutaminyl-L-prolyl-L-leucyl-L- arginyl-N-methylglycyl-L-lysinamide] cyclic (6-15),(6'-15')-bis(disulfide) |

In various hormonal disorders :

| | |
|---|---|
| COR-005 | cyclo(-y-aminobutyryl-L-phenylalanyl-L-tryptophanyl-D-tryptophanyl-L-lysyl-L-threonly-L-phenylalanyl-N-3-carboxypropyl)-glycine amide, acetate salt |
| Etelcalcetide Hydrochloride | CARRRAR (SEQ ID NO: 66) |
| Gonadorelin Acetate | acetic acid;(2S)-N-[(2S)-1-[[(2S)-1-[[(2S)-1-[[(2S)-1-[[2-[[(2S)-1-[[(2S)-1-[(2S)-2-[(2-amino-2-oxoethyl)carbamoyl]pyrrolidin-1-yl]-5-(diaminomethylideneamino)-1-oxopentan-2-yl]amino]-4-methyl-1-oxopentan-2-yl]amino]-2-oxoethyl]amino]-1-oxo-3-phenylpropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide;hydrate |
| histrelin | (2S)-N-[(2S)-1-[[(2S)-1-[[(2S)-1-[[(2S)-1-[[(2R)-3-(1-benzylimidazol-4-yl)-1-[[(2S)-1-[[(2S)-5-(diaminomethylideneamino)-1-[(2S)-2-(ethylcarbamoyl)pyrrolidin-1-yl]-1-oxopentan-2-yl]amino]-4-methyl-1-oxopentan-2-yl]amino]-1-oxopropan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide |
| Lanreotide Acetate | acetic acid; (4R, 7S, 10S, 13R, 16S, 19R)-10-(4-aminobutyl)-N-[(2S,3R)-1-amino-3-hydroxy-1-oxobutan-2-yl]-19-[[(2R)-2-amino-3-naphthalen-2-ylpropanoyl]amino]-16-[(4-hydroxyphenyl)methyl]-13-(1H-indol-3-ylmethyl)-6,9,12,15,18-pentaoxo-7-propan-2-yl-1,2-dithia-5,8,11,14,17-pentazacycloicosane-4-carboxamide |
| MOD-4023 | MATGSRTSLLLAFGLLCLPWLQEGSASSSSKAPPPSLPSPSRLPGPS DTPILPQFPTIPLSRLFDNAMLRAHRLHQLAFDTYQEFEEAYIPKEQK YSFLQNPQTSLCFSESIPTPSNREETQQKSNLELLRISLLLIQSWLEPV QFLRSVFANSLVYGASDSNVYDLLKDLEEGIQTLMGRLEDGSPRTG QIFKQTYSKFDTNSHNDDALLKNYGLLYCFRKDMDKVETFLRIVQCR SVEGSCGFSSSSKAPPPSLPSPSRLPGPSDTPILPQSSSSKAPPPSL PSPSRLPGPSDTPILPQ (SEQ ID NO: 67) |
| Octreotide Acetate Long Acting | acetic acid;10-(4-aminobutyl)-19-[(2-amino-3-phenylpropanoyl)amino]-16-benzyl-N-(1,3-dihydroxybutan-2-yl)-7-(1-hydroxyethyl)-13-(1H-indol-3-ylmethyl)-6,9,12,15,18-pentaoxo-1,2-dithia-5,8,11,14,17-pentazacycloicosane-4-carboxamide |
| Pasireotide ER | [(20R)-9-(4-aminobutyl)-3-benzyl-12-(1H-indol-3-ylmethyl)-2,5,8,11,14,17-hexaoxo-15-phenyl-6-[(4-phenylmethoxyphenyl)methyl]-1,4,7,10,13,16-hexazabicyclo[16.3.0]henicosan-20-yl] N-(2-aminoethyl)carbamate |
| Somatrem | Somatotropin (human) |
| Triptorelin | N-[1-[[1-[[1-[[1-[[1-[[1-[[1-[2-[(2-amino-2-oxoethyl)carbamoyl]pyrrolidin-1-yl]-5-(diaminomethylideneamino)-1-oxopentan-2-yl]amino]-4-methyl-1-oxopentan-2-yl]amino]-3-(1H-indol-3-yl)-1-oxopropan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide |
| Triptorelin Pamoate | (2S)-N-[(2S)-1-[[(2S)-1-[[(2S)-1-[[(2S)-1-[[(2R)-1-[[(2S)-1-[[(2S)-1-[(2S)-2-[(2-amino-2-oxoethyl)carbamoyl]pyrrolidin-1-yl]-5-(diaminomethylideneamino)-1-oxopentan-2-yl]amino]-4-methyl-1-oxopentan-2-yl]amino]-3-(1H-indol-3-yl)-1-oxopropan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide;4-[(3-carboxy-2-hydroxynaphthalen-1-yl)methyl]-3-hydroxynaphthalene-2-carboxylic acid |

In various immunological disorders :

| | |
|---|---|
| Aclerastide | 1-7-Angiotensin II, 3-L-norleucine-5-L-isoleucine |
| ATI-2341 | MGYQKKLRSMTDKYRL (SEQ ID NO: 68) |
| Dalazatide | o-PHOSPHONO-L-Tyrosyl-2-(2-(2-aminoethoxy)ethoxy)acetyl(potassium channel toxin kappa-stichotoxin-shela stoichactis helianthus (caribbean sea anemone)) peptidamide |
| Disitertide | (2S)-4-amino-2-[[(2S)-5-amino-2-[[(2S)-2-[[(2S)-2-[[(2S)-2-[[(2S)-2-[[(2S,3S)-2-[[(2S,3S)-2-[[(2S)-2-[[(2S)-2-[[(2S)-2-[[(2S)-2-[[(2S)-2-[[(2S,3R)-2-amino-3-ydroxybutanoyl]amino]-3-hydroxypropanoyl]amino]-4-methylpentanoyl]amino]-3-carboxypropanoyl]amino]propanoyl]amino]-3-hydroxypropanoyl]amino]-3-methylpentanoyl]amino]-3-thylpentanoyl]amino]-3-(1H-indol-3-yl)propanoyl]amino]propanoyl]amino]-4-methylsulfanylbutanoyl]amino]-4-methylsulfanylbutanoyl]amino]-5-oxopentanoyl]amino]-4-oxobutanoic acid |
| Edratide | GYYWSWIRQPPGKGEEWIG (SEQ ID NO: 69) |
| Forigerimod Acetate | RIHMVYSKRXGKPRGYAFIEY, acetate X : DL-Threonine (SEQ ID NO: 70) |
| hPTH-1-37 | SVSEIQLMHNLGKHLNSMERVEWLRKKLQDVHNFVAL ((SEQ ID NO: 71) |
| Icatibant Acetate | (2S)-2-[[(3aS,7aS)-1-[(3R)-2-[(2S)-2-[[(2S)-2-[[2-[[(2S,4R)-1-[(2S)-1-[(2S)-2-[[(2R)-2-amino-5-(diaminomethylideneamino)pentanoyl]amino]-5-(diaminomethylideneamino)pentanoyl]pyrrolidine-2-carbonyl]-4-hydroxypyrrolidine-2-carbonyl]amino]acetyl]amino]-3-thiophen-2-ylpropanoyl]amino]-3-hydroxypropanoyl]-3,4-dihydro-1H-isoquinoline-3-carbonyl]-2,3,3a,4,5,6,7,7a-octahydroindole-2-carbonyl]amino]-5-(diaminomethylideneamino)pentanoic acid;acetic acid |

In various infectious diseases :

| | |
|---|---|
| ALB-408 | LVRYTKKVPQVSTPTL (SEQ ID NO: 72) |
| Enfuvirtide | YTSLIHSLIEESQNQQEKNEQELLELDKWASLWNWF (SEQ ID NO: 73) |
| FB-006M | WEEWDREINNYTKLIHELIEESQNQQEKNEQELL (SEQ ID NO: 74) |
| LTX-109 | (2S)-2-amino-5-(diaminomethylideneamino)-N-[(2S)-1-[[(2S)-5-(diaminomethylideneamino)-1-oxo-1-(2-phenylethylamino)pentan-2-yl]amino]-1-oxo-3-(2,5,7-tritert-butyl-1H-indol-3-yl)propan-2-yljpentanamide |
| NP-213 | 2-[3-[(2S,5S,8S,11S,14S,17S,20S)-5,8,11,14,17,20-hexakis[3-(diaminomethylideneamino)propyl]-3,6,9,12,15,18,21-heptaoxo-1,4,7,10,13,16,19-heptazacyclohenicos-2-yl]propyl]guanidine |
| Thymalfasin | SDAAVDTSSEITTKDLKEKKEVVEEAEN (SEQ ID NO: 75) |
| Pidotimod | (4R)-3-[(2S)-5-oxopyrrolidine-2-carbonyl]-1,3-thiazolidine-4-carboxylic acid |
| Sifuvirtide | SWETWEREIENYTRQIYRILEESQEQQDRNERDLLE (SEQ ID NO: 76) |
| Thymalfasin | SDAAVDTSSEITTKDLKEKKEVVEEAEN (SEQ ID NO: 77) |
| VIR-576 | LEAIPCSIPPEFLFGKPFVFLEAIPCSIPPEFLFGKPFVF (SEQ ID NO: 78) |

In various male health diseases :

| Chorionic Gonadotrophin | Gonadotrophin |
|---|---|
| TAK-448 | 2-(N-Acetyl-d-tyrosyl-trans-4-hydroxy-l-prolyl-l-asparaginyl-l-threonyl-l-phenylalanyl)hydrazinocarbonyl-l-leucyl-Nω-methyl-l-arginyl-1-tryptophanamide monoacetate |

In various metabolic disorders :

| | |
|---|---|
| abaloparatide | C2.29-methyl(22-L-glutamic acid(F>E),23-L-leucine(F>L),25-L-glutamic acid(H>E),26-L-lysine(H>K),28-L-leucine(I>L),30-L-lysine(E>K),31-L-leucine(I>L))human parathyroid hormone-related protein-(1-34)-proteinamide |
| AC-163794 | EGTFISDYSIAMDKIHQQDFVNWLLAQKPSSGAPPPS (SEQ ID NO: 79) |
| Albenatide | S3.34-{1-[(23S)-23-{[exendin-4 Heloderma suspectum precursor-(48-86)-peptidyl (exenatidyl)]amino}-3,12,24-trioxo-7,10-dioxa-4,13,18,25-tetraazapentacosyl]-2,5-dioxopyrrolidin-3-yl}human serum albumin |
| AMG-5041 | (2S)-2-[[(2S,3R)-2-[[2-[[(2S)-5-amino-2-[[(2S)-2-[[(2S)-2-amino-3-(1H-imidazol-5-yl)propanoyl]amino]-3-hydroxypropanoyl]amino]-5-oxopentanoyl]amino]acetyl]amino]-3-hydroxybutanoyl]amino]-3-phenylpropanoic acid |
| Argipressin | 1-[19-amino-7-(2-amino-2-oxoethyl)-10-(3-amino-3-oxopropyl)-13-benzyl-16-[(4-hydroxyphenyl)methyl]-6,9,12,15,18-pentaoxo-1,2-dithia-5,8,11,14,17-pentazacycloicosane-4-carbonyl]-N-[1-[(2-amino-2-oxoethyl)amino]-5-(diaminomethylideneamino)-1-oxopentan-2-yl]pyrrolidine-2-carboxamide |
| Calcitonin | CGNLSTCMLGTYTQDFNKFHTFPQTAIGVGAP (SEQ ID NO: 80) |
| DD-04107 | palmitoyl-EEMQRR (SEQ ID NO: 81) |
| Efpeg lenatide | (2S)-1-[3-[2-[3-[[(5S)-5-amino-5-carboxypentyl]amino]propoxy]ethoxy]propyl]pyrrolidine-2-carboxylic acid |
| Exenatide | Exendin 3 (Heloderma horridum), 2-glycine-3-L-glutamic acid |
| Exendin-(9-39) | DLSKQMEEEAVRLFIEWLKNGGPSSGAPPPS (SEQ ID NO: 82) |
| HIP-2B | IGLHDPTQGTEPNG (SEQ ID NO: 83) |
| HS-20004 | EGTFTSDVSSYLEEEAAKEFIAWLVRGGPSSGAPPPS (SEQ ID NO: 84) |
| insulin aspart | FVNQHLC₂GSHLVEALYLVC₃GERGFFYTDKT, GIVEQC₁C₂TSIC₁SLYQLENYC₃N (SEQ ID NO: 85) Two chains linked by disulfid bonds between two C₁, C₂ or C₃ |
| insulin degludec | FVNQHLC₂GSHLVEALYLVC₃GERGFFYTPK, GIVEQC₁C₂TSIC₁SLYQLENYC₃N (SEQ ID NO: 86) Two chains linked by disulfid bonds between two C₁, C₂ or C₃ |
| insulin detemir | FVNQHLC₂GSHLVEALYLVC₃GERGFFYTPK (myristoyl), GIVEQC₁C₂TSIC₁SLYQLENYC₃N (SEQ ID NO: 87) Two chains linked by disulfid bonds between two C₁, C₂ or C₃ |
| insulin glargine | FVNQHLC₂GSHLVEALYLVC₃GERGFFYTPKTRR, GIVEQC₁C₂TSIC₁SLYQLENYC₃G (SEQ ID NO: 88) Two chains linked by disulfid bonds between two C₁, C₂ or C₃ |
| insulin glulisine | FVKQHLC₂GSHLVEALYLVC₃GERGFFYTPET, GIVEQC₁C₂TSIC₁SLYQLENYC₃N (SEQ ID NO: 89) Two chains linked by disulfid bonds between two C₁, C₂ or C₃ |
| insulin lispro | FVNQHLC₂GSHLVEALYLVC₃GERGFFYTKPT, GIVEQC₁C₂TSIC₁SLYQLENYC₃N (SEQ ID NO: 90) Two chains linked by disulfid bonds between two C₁, C₂ or C₃ |
| insulin neutral | FVNQHLC₂GSHLVEALYLVC₃GERGFFYTPKA, GIVEQC₁C₂TSIC₁SLYQLENYC₃N (SEQ ID NO: 91) |
| | Two chains linked by disulfid bonds between two C₁, C₂ or C₃ |
| insulin zinc | Insulin protamine zinc |
| liraglutide (recombinant) | N26-(hexadecanoyl-gamma-glutamyle)-[34-arginine]GLP-1-(7-37)-peptide |
| Lixisenatide | HGEGTFTSDLSKQMEEEAVRLFIEWLKNGGPSSGAPPSKKKKKK (SEQ ID NO: 92) |
| NN-9535 | Glycine, L-histidyl-2-methylalanyl-L-alpha-glutamylglycyl-L-threonyl-L-phenylalanyl-L- threonyl-L-seryl-L-alpha-aspartyl-L-valyl-L-seryl-L-seryl-L-tyrosyl-L-leucyl-L-alpha-glutamylglycyl- L-glutaminyl-L-alanyl-L-alanyl-N6-[N-(17-carboxy-1-oxoheptadecyl-L-gamma-glutamyl[2-(2-aminoethoxy)ethoxy]acetyl[2-(2-aminoethoxy)ethoxy]acetyl]-L-lysyl-L-alpha-glutamyl-L- phenylalanyl-L-isoleucyl-L-alanyl-L-tryptophyl-L-leucyl-L-valyl-L-arginylglycyl-L-arginyl- |
| NN-9536 | Glycine, L-histidyl-2-methylalanyl-L-alpha-glutamylglycyl-L-threonyl-L-phenylalanyl-L- threonyl-L-seryl-L-alpha-aspartyl-L-valyl-L-seryl-L-seryl-L-tyrosyl-L-leucyl-L-alpha-glutamylglycyl- L-glutaminyl-L-alanyl-L-alanyl-N6-[N-(17-carboxy-1-oxoheptadecyl-L-gamma-glutamyl[2-(2-aminoethoxy)ethoxy]acetyl[2-(2-aminoethoxy)ethoxy]acetyl]-L-lysyl-L-alpha-glutamyl-L- phenylalanyl-L-isoleucyl-L-alanyl-L-tryptophyl-L-leucyl-L-valyl-L-arginylglycyl-L-arginyl- |
| OG-217SC | Glycine, L-histidyl-2-methylalanyl-L-alpha-glutamylglycyl-L-threonyl-L-phenylalanyl-L-threonyl-L-seryl-L-alpha-aspartyl-L-valyl-L-seryl-L-seryl-L-tyrosyl-L-leucyl-L-alpha-glutamylglycyl- L-glutaminyl-L-alanyl-L-alanyl-N6-[N-(17-carboxy-1-oxoheptadecyl-L-gamma-glutamyl[2-(2-aminoethoxy)ethoxy]acetyl[2-(2-aminoethoxy)ethoxy]acetyl]-L-lysyl-L-alpha-glutamyl-L- phenylalanyl-L-isoleucyl-L-alanyl-L-tryptophyl-L-leucyl-L-valyl-L-arginylglycyl-L-arginyl- |
| Pasireotide | [(3S,6S,9S,12R,15S,18S,20R)-9-(4-aminobutyl)-3-benzyl-12-(1H-indol-3-ylmethyl)-2,5,8,11,14,17-hexaoxo-15-phenyl-6-[(4-phenylmethoxyphenyl)methyl]-1,4,7,10,13,16-hexazabicyclo[16.3.0]henicosan-20-yl] N-(2-aminoethyl)carbamate |
| PEX-168 | HGEGTFTSDLSKQMEEEAVRLFIEWLKNGGPSSGAPPPS (PEG) (SEQ ID NO: 93) |
| Pexiganan | GIGKFLKKAKKFGKAFVKLLKK (SEQ ID NO: 94) |
| PI-406 | Insulin (human), 28B-L-aspartic acid- |
| PMZ-2123 | DPVLW (SEQ ID NO: 95) |
| pramlintide | Amylin (human), 25-L-proline-28-L-proline-29-L-proline- |
| protamine zinc insulin | Insulin protamine zinc |
| PT-302 | l-histidylglycyl-l-glutamylglycyl-l-threonyl-l-phenylalanyl-l-threonyl-l-seryl-l-aspartyl-l-leucyl-l-seryl-l-lysyl-l-glutaminyl-l-methionyl-l-glutamyl-l-glutamyl-lglutamyl-l-alanyl-l-valyl-l-arginyl-l-leucyl-l-phenylalanyl-l-isoleucyl-l-glutamyl-l-tryptophyl-l-leucyl-l-lysyl-l-asparaginylglycylglycyl-l-prolyl-l-seryl-l-serylglycyl-l-alanyl-l-prolyl-l-prolyl-l-prolyl-l-serinamide |
| Saxenda | N26-(hexadecanoyl-gamma-glutamyle)-[34-arginine]GLP-1-(7-37)-peptide |
| Teriparatide | SVSEIQLMHNLGKHLNSMERVEWLRKKLQDVHNF (SEQ ID NO: 96) |
| teriparatide acetate | SVSEIQLMHNLGKHLNSMERVEWLRKKLQDVHNF, acetate (SEQ ID NO: 97) |
| tesamorelin acetate | N-[(3E)-1-oxo-3-hexenyl]-YADAIFTNSYRKLGQLSARKLLQDIMSRQQGESNQERGARARL, acetate (SEQ ID NO: 98) |
| Toujeo | 21(sup A)-Glycine-30(sup B)a-L-arginine-30(sup B)b-L-arginine insulin (human) |
| VTC-G 15 | LVKGR (SEQ ID NO: 99) |
| ZP-4207 | HSQGTFTSDYSKYLDRARADDFVAWLKST (SEQ ID NO: 100) |

In various musculoskeletal disorders :

| | |
|---|---|
| Vosoritide | L-prolylglycyl-(human C-type natriuretic peptide-(17-53)-peptide (CNP-37)), cyclic-(23-39)-disulfide |

In various nutritional disorders :

| | |
|---|---|
| parathyroid hormone | (2S)-2-amino-3-[3-(hydroxymethyl)-4-phosphonooxyphenyl]propanoic acid |

In various oncological disorders :

| | |
|---|---|
| abarelix | (2R)-2-[[(2S)-2-[[(2S)-2-[[(2R)-2-[[(2R)-2-[[(2R)-2-acetamido-3-naphthalen-2-ylpropanoyl]amino]-3-(4-chlorophenyl)propanoyl]amino]-3-pyridin-3-ylpropanoyl]amino]-3-hydroxypropanoyl]-methylamino]-3-(4-hydroxyphenyl)propanoyl]amino]-N-[(2S)-1-[[(2S)-1-[(2S)-2-[[(2R)-1-amino-1-oxopropan-2-yl]carbamoyl]pyrrolidin-1-yl]-1-oxo-6-(propan-2-ylamino)hexan-2-yl]amino]-4-methyl-1-oxopentan-2-yl]butanediamide |
| AD-01 | QIRQQPRDPPTETLELEVSPDPAS (SEQ ID NO: 101) |
| Adipotide | CKGGRAKDCGGKLAKLAKKLAKLAK (SEQ ID NO: 102) |
| Antineoplaston Therapy | Antineoplaston A10: Benzeneacetamide, N-(2,6-dioxo-3-piperidinyl)-, (S)- Antineoplaston AS2-1: L-Glutamine, N2-(phenylacetyl)-, monosodium salt, mixt. with sodium benzeneacetate |
| ATSP-7041 | LTFEYWAQXSAA (X : Cba) (SEQ ID NO: 103) |
| BMTP-11 | CGRRAGGSCGGDKLAKLAKKLAKLAK (SEQ ID NO: 104) |
| Box-5 | MDGCEL (SEQ ID NO: 105) |
| Buserelin Acetate | acetic acid;(2S)-N-[(2S)-1-[[(2S)-1-[[(2S)-1-[[(2S)-1-[[(2R)-1-[[(2S)-1-[[(2S)-5-(diaminomethylideneamino)-1-[(2S)-2-(ethylcarbamoyl)pyrrolidin-1-yl]-1-oxopentan-2-yl]amino]-4-methyl-1-oxopentan-2-yl]amino]-3-[(2-methylpropan-2-yl)oxy]-1-oxopropan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide |
| CBP-501 | D-Arginine, 4-benzoyl-D-phenylalanyl-D-seryl-D-tryptophyl-D-seryl-2,3,4,5,6-pentafluoro-D-phenylalanyl-3-cyclohexyl-D-alanyl-D-arginyl-D- arginyl-D-arginyl-D-glutaminyl-D-arginyl |
| CMS-024 | LYS |
| Degarelix Acetate | D-Alaninamide, N-acetyl-3-(naphtalen-2-yl)-D-alanyl-4-chloro-D-phenylalanyl-3-(pyridin-3-yl)-D-alanyl-L-seryl-4-((((4S)-2,6-dioxohexahydropyrimidin-4-yl)carbonyl)amino)-L-phenylalanyl-4-(carbamoylamino)-D-phenylalanyl-L-leucyl-N6-(1-methylethyl)-L-lysyl-L-prolyl-, acetate |
| Dolcanatide | NDECELCVNVACTGCL (SEQ ID NO: 106) |
| Dusquetide | RIVPA (SEQ ID NO: 107) |
| Edotreotide | 2-[4-[2-[[(2R)-1-[[(4R,7S,10S,13R,16S,19R)-10-(4-aminobutyl)-4-[[(2R,3R)-1,3-dihydroxybutan-2-yl]carbamoyl]-7-[(1R)-1-hydroxyethyl]-16-[(4-hydroxyphenyl)methyl]-13-(1H-indol-3-ylmethyl)-6,9,12,15,18-pentaoxo-1,2-dithia-5,8,11,14,17-pentazacycloicos-19-yl]amino]-1-oxo-3-phenylpropan-2-yl]amino]-2-oxoethyl]-7,10-bis(carboxymethyl)-1,4,7,10-tetrazacyclododec-1-yl]acetic acid |
| EPI-506 | 3-[4-[2-[4-(3-chloro-2-hydroxypropoxy)phenyl]propan-2-yl]phenoxy]propane-1,2-diol |
| Foxy-5 | MDGCEL (SEQ ID NO: 108) |
| GO-2032c | RRRRRRRRRCQCRRKN (SEQ ID NO: 109) |
| Goserelin | (2S)-N-[(2S)-1-[[(2S)-1-[[(2S)-1-[[(2S)-1-[[(2R)-1-[[(2S)-1-[[(2S)-1-[(2S)-2-[(carbamoylamino)carbamoyl]pyrrolidin-1-yl]-5-(diaminomethylideneamino)-1-oxopentan-2-yl]amino]-4-methyl-1-oxopentan-2-yl]amino]-3-[(2-methylpropan-2-yl)oxy]-1-oxopropan-2-yl]amino]-3-(4-hydroxyphenyl)-1-oxopropan-2-yl]amino]-3-hydroxy-1-oxopropan-2-yl]amino]-3-(1H-indol-3-yl)-1-oxopropan-2-yl]amino]-3-(1H-imidazol-5-yl)-1-oxopropan-2-yl]-5-oxopyrrolidine-2-carboxamide |
| LTX-315 | KKWWKKWXK; X : Dip (SEQ ID NO: 110) |
| LY-2510924 | FYKRXGEK; X : Nal (SEQ ID NO: 111) |
| Ozarelix | (2S)-1-[(2S)-2-[[(2S)-2-[[(2R)-2-[[(2S)-2-[[(2S)-2-[[(2R)-2-[[(2R)-2-[[(2R)-2-acetamido-3-naphthalen-2-ylpropanoyl]amino]-3-(4-chlorophenyl)propanoyl]amino]-3-pyridin-3-ylpropanoyl]amino]-3-hydroxypropanoyl]-methylamino]-3-(4-hydroxyphenyl)propanoyl]amino]-6-(carbamoylamino)hexanoyl]amino]hexanoyl]amino]-5-(diaminomethylideneamino)pentanoyl]-N-[(2R)-1-amino-1-oxopropan-2-yl]pyrrollidine-2-carboxamide |
| Plitidepsin | (2S)-N-[(2R)-1-[[(3S,6R,8S,12S,16R,17S, 23S)-13-[(2S)-butan-2-yl]-12-hydroxy-20-[(4-methoxyphenyl)methyl]-6,17, 21-trimethyl-3-(2-methylpropyl)-2,5,7,10,15,19, 22-heptaoxo-8-propan-2-yl-9,18-dioxa-1,4,14, 21-tetrazabicyclo[21.3.0]hexacosan-16-yl]amino]-4-methyl-1-oxopentan-2- yl]-N-methyl-1-(2- oxopropanoyl)pyrrolidine-2-carboxamide |
| Romidepsin | (1S,7Z,10S,16E,21R)-7-ethylidene-4,21-di(propan-2-yl)-2-oxa-12,13-dithia-5,8,20,23-tetrazabicyclo[8.7.6]tricos-16-ene-3,6,9,19,22-pentone |
| SORC-13 | KIEFLHPSKVDLPR (SEQ ID NO: 112) |
| SPI-1620 | L-Tryptophan, N-(3-carboxy-1-oxopropyl)-L-alanyl-L-alpha-glutamyl-L-alpha-glutamyl-L-alanyl-L-valyl-L-tyrosyl-L-tyrosyl-L-alanyl-L-histidyl-L-leucyl-L-alpha-aspartyl-L-isoleucyl-L-isoleucyl- |

In various ophthalmological disorders :

| | |
|---|---|
| B27-PD | ALNEDLSSWTAADT (SEQ ID NO: 113) |
| C-16Y | DFKLFAVYIKYR (SEQ ID NO: 114) |
| RGN-259 | MSDKPDMAEIEKFDKSKLKKTETQEKNPLPSKETIEQEKQAGES (SEQ ID NO: 115) |
| Spaglumic Acid | (2S)-2-[[(2S)-2-acetamido-3-carboxypropanoyl]amino]pentanedioic acid |

In various functional gastrointestinal disorders :

| | |
|---|---|
| Nepadutant | (2S)-2-[[(3S,6S,9S,12S)-12-[[(2S)-4-[[(2R,3R,4R,5S,6R)-3-acetamido-4, 5-dihydroxy-6-(hydroxymethyl)oxan-2-yl]amino]-2-amino-4-oxobutanoyl]aminol-6-benzyl-9-(1H-indol-3-ylmethyl)-5,8,11,14-tetraoxo-1,4,7,10-tetrazacyclotetradecane-3-carbonyl]amino]-4-methylpentanoic acid |

| In various respiratory disorders : | |
|---|---|
| CGEN-25009 | GQKGQVGPPGAAVRRAYAAFSVGRRAYAAFSV (SEQ ID NO: 116) |
| Lucinactant | KLLLLKLLLLKLLLLKLLLLK-surfactant (SEQ ID NO: 117) |
| MMI-0100 | YARAAARQARAKALARQLGVAA (SEQ ID NO: 118) |

| In various women's health disorders : | |
|---|---|
| Atosiban | (2S)-N-[(2S)-5-amino-1-[(2-amino-2-oxoethyl)amino]-1-oxopentan-2-yl]-1-[(4R,7S,13S,16R)-7-(2-amino-2-oxoethyl)-13-[(2S)-butan-2-yl]-16-[(4-ethoxyphenyl) methyl]-10-[(1R)-1-hydroxyethyl]-6,9,12,15,18-pentaoxo-1,2-dithia-5,8,11,14,17-pentazacycloicosane-4-carbonyl]pyrrolidine-2-carboxamide |
| Barusiban | (4S,7S,10S,16R)-N-[(2S)-5-amino-1-hydroxypentan-2-yl]-7-(2-amino-2-oxoethyl)-13-[(2S)-butan-2-yl]-10-[(2R)-butan-2-yl]-16-(1H-indol-3-ylmethyl)-N-methyl-6,9,12,15,18-pentaoxo-1-thia-5,8,11,14,17-pentazacycloicosane-4-carboxamide |
| Bremelanotide | L-Lysine, N-acetyl-L-norleucyl-L-a-aspartyl-L-histidyl-D-phenylalanyl-L-arginyl-L-tryptophyl-, (2-7)-lactam |
| Carbetocin | N-[1-[(2-amino-2-oxoethyl)amino]-4-methyl-1-oxopentan-2-yl]-1-[6-(2-amino-2-oxoethyl)-9-(3-amino-3-oxopropyl)-12-butan-2-yl-15-[(4-methoxyphenyl)methyl]-5,8,11,14,17-pentaoxo-1-thia-4,7,10,13,16-pentazacycloicosane-3-carbonyl]pyrrolidine-2-carboxamide |
| Cetrorelix | (2S)-1-[(2S)-2-[[(2S)-2-[[(2R)-2-[[(2S)-2-[[(2S)-2-[[(2R)-2-[[(2R)-2-[[(2R)-2-acetamido-3-naphthalen-2-ylpropanoyl]amino]-3-(4-chlorophenyl)propanoyl]amino]-3-pyridin-3-ylpropanoyl]amino]-3-hydroxypropanoyl]amino]-3-(4-hydroxyphenyl)propanoyl]amino]-5-(carbamoylamino)pentanoyl]amino]-4-methylpentanoyl]amino]-5-(diaminomethylideneamino)pentanoyl]-N-[(2R)-1-amino-1-oxopropan-2-yl]pyrrolidine-2-carboxamide |
| Cetrorelix Acetate | (2S)-1-[(2S)-2-[[(2S)-2-[[(2R)-2-[[(2S)-2-[[(2S)-2-[[(2R)-2-[[(2R)-2-[[(2R)-2-acetamido-3-naphthalen-2-ylpropanoyl]amino]-3-(4-chlorophenyl)propanoyl]amino]-3-pyridin-3-ylpropanoyl]amino]-3-hydroxypropanoyl]amino]-3-(4-hydroxyphenyl)propanoyl]amino]-5-(carbamoylamino)pentanoyl]amino]-4-methylpentanoyl]amino]-5-(diaminomethylideneamino)pentanoyl]-N-[(2R)-1-amino-1-oxopropan-2-yl]pyrrolidine-2-carboxamide;acetic acid |
| Follitropin | 1-[19-amino-7-(2-amino-2-oxoethyl)-13-butan-2-yl-10-(1-hydroxyethyl)-16-[(4-hydroxyphenyl)methyl]-6,9,12,15,18-pentaoxo-1,2-dithia5,8,11,14,17-pentazacycloicosane-4-carbonyl]-N-[1-[(2-amino-2-oxoethyl)amino]-4-methyl-1-oxopentan-2-yl]pyrrolidine-2-carboxamide |
| Ganirelix acetate | (2S)-1-[(2S)-2-[[(2S)-2-[[(2R)-2-[[(2S)-2-[[(2S)-2-[[(2R)-2-[[(2R)-2-[[ (2R)-2-acetamido-3-naphthalen-2-ylpropanoyl]amino]-3-(4-chlorophenyl) propanoyl]amino]-3-pyridin-3-ylpropanoyl]amino]-3-hydroxypropanoyl] amino]-3-(4-hydroxyphenyl)propanoyl]amino]-6-[bis(ethylamino) methylideneamino]hexanoyl]amino]-4-methylpentanoyl]amino]-6-[bis (ethylamino)methylideneamino]hexanoyl]-N-[(2R)-1-amino-1-oxopropan-2-yl] pyrrolidine-2-carboxamide; acetic acid |
| Gonadorelin | XHWSYGLRPG X : Pyroglutamic acid (SEQ ID NO: 119) |
| Merotocin | Glycinamide, N-(4-mercapto-1-oxobutyl)-L-tyrosyl-L-isoleucyl-L-glutaminyl-L-asparaginyl-L-cysteinyl-N-((4-fluorophenyl)methyl)glycyl-L-leucyl-, cyclic (1->5)-thioether |
| Nafarelin | X₁HWSYXLRPG (SEQ ID NO: 120) X₁: Pyroglutamic acid, X : Nal |
| Oxytocin | CYIQNCPLG (SEQ ID NO: 121) |
| Urofollitropin | CYITNCPLG (SEQ ID NO: 122) |

The present invention will be further illustrated by the following examples. However these examples should not be interpreted in any way as limiting the scope of the present invention.

### EXAMPLES

### EXAMPLE 1 : GENERAL MATERIAL AND METHODS

Reagents were obtained from commercial source and used without any further purification. Fmoc-L-amino acids were purchased from Novabiochem, Polypeptides and Iris Biotech. Fmoc-protected Rink Amide NovaGel® resin was purchased from Novabiochem and the overall yields for the solid-phase syntheses were calculated based on the initial loadings provided by the supplier (0.7 mmol/g). Fmoc-protected Wang NovaGel® resin was purchased from Novabiochem and the overall yields for the solid-phase syntheses were calculated based on the initial loadings provided by the supplier (0.1 mmol/g).

### Analytical reverse-phase high performance liquid chromatography (RP-HPLC) analysis

Analysis were performed either on a C18 Sunfire column (5µm, 4.6mm x 150mm) using a linear gradient (5% to 95% in 20min, flow rate of 1mL.min⁻¹) of solvent B (0.1 % TFA in CH₃CN, v/v) in solvent A (0.1 % TFA in H₂O, v/v). Detection was set AT 220nm and 254nM.

### Semi-preparative RP-HPLC chromatography purifications

Purifications were performed on Sunfire C18 column (5µm, 19 x 150mm) on Gilson PLC2020 with absorption detection. The separation was achieved using successive isocratic and linear gradients (5min at 5%; 5% to 60% in 30min; 60% to 100% in 10min; flow rate of 20mL.min⁻¹) of solvent B (0.1% TFA in CH₃CN, v/v) in solvent A (0.1% TFA in H₂O, v/v).

### Liquid chromatography mass spectra (LC-MS) analysis

Analysis were obtained on a ZQ (Z quadripole) Waters/Micromass spectrometer equipped with an X-Terra C18 column (0.5µm, 4.6mm x 50mm) using electrospray ionization mode (ESI).

### High resolution mass spectra (HR-MS) analysis

Analysis were acquired on a Bruker MicroTof mass spectrometer, using electrospray ionization (ESI) and a time-of-flight analyzer (TOF) or on an Autoflex II TOF/TOF Bruker mass spectrometer using matrix-assisted laser desorption/ionization technique (MALDI) and a time-of-light analyzer (TOF).

### General protocol for standard automated solid-phase peptide synthesis (SPPS)

Standard automated solid-phase peptide synthesis (SPPS) were performed on an Applied Biosystem ABI 433A synthesizer (Appelar, France). The elongation was carried out by coupling a 10-fold excess of Fmoc-L-amino acid derivatives, using 2-(1H-benzotriazol-1-yl)-1,1,3,3-tetramethyluronium hexafluorophosphate (HBTU), 1-hydroxybenzotriazole (HOBt), and diisopropylethylamine (Hünig's base) (DIPEA) as coupling reagents in N,N-dimethylformamide (DMF) as solvent. At the end of the peptide sequence synthesis, the resin was washed with CH₂Cl₂ and MeOH and then dried *in vacuo.* After each coupling step, Fmoc deprotection was performed by treatment with piperidine monitored by UV at 301 nm.

### General protocol for peptide elongation by manual SPPS

Peptide elongation was performed starting from fried resin previously synthetized by standard automated SPPS. Non-automated SPPS were performed in polypropylene tubes equipped with polyethylene frits and polypropylene caps using an orbital agitator shaking device.

The Fmoc-protected resin (1 equiv) was swollen 1h in DMF and the excess solvent was removed by filtration. Cleavage of the Fmoc protecting group was performed in a solution of 20% (v/v) piperidine in DMF (2 times for 15min). The piperidine solution was drained off and the resin was washed with successively DMF, CH₂Cl₂ and MeOH (3x0.5mL).

All Fmoc-protected amino acids (4 equiv) were coupled in N,N-dimethylformamide (DMF) for 45min using HBTU (3.8 equiv) and HOBt (4 equiv) with N,N-diisopropylethylamine (DIEA) (12 equiv) as activating agents. The excess solvent was removed by filtration and the resin was washed with successively DMF, CH₂Cl₂ and MeOH (3x0.5mL).

The cycle of coupling, washing and deprotection were repeated until the targeted peptides were obtained. The completion of couplings and Fmoc deprotections were monitored with ninhydrin test and TNBS test:
- Ninhydrin test (for primary amines) : Resin beads were suspended in 2 drops of a solution containing 5g of ninhydrin dissolved in 100mL of ethanol, 2 drops of a solution containing 80g of liquefied phenol in 20mL of ethanol, and 2 drops of a 0.001 M aqueous solution of potassium cyanide to 98mL pyridine. The mixture was heated at 100°C for 1min. The color positive test (presence of free amino groups). A yellow or blue solution and yellow beads indicate a negative test.
- TNBS test (for primary amines): Resin beads were suspended in 2 drops of a solution containing 10% (v/v) DIPEA in DMF and 2 drops of a solution containing 2,4,6-trinitrobenzenesulfonic acid (TNBS) in DMS. The color of the solution and the beads were observed. A yellow-red solution and red beads indicate a positive test. A yellow-red solution and yellow beads indicate a negative test.

### General protocol for peptide elongation with a perfluoroalkyl chain

The resin containing the peptide sequence of interest (1 equiv) was swollen in DMF, and the excess solvent was removed by filtration. A solution of piperidine in DMF (20% v/v - 0.5mL) was added, and the mixture was shaken at room temperature for 15min. The solution was drained, and the operation was repeated for 15min. The solution was drained, and the resin was washed with DMF and CH₂Cl₂. In a separate vial, DIEA (8 equiv) was added to a solution of 4,4,5,5,6,6,7,7,8,8,9,9,10,10,11,11-heptadecafluoroundecanoic acid (2 equiv), HBTU (2 equiv), and HOBt (1.9 equiv) in DMF (0.5mL). The mixture was stirred at room temperature for 1 min and was added to the resin. The mixture was shaken at room temperature for 2h. The solution was drained and the resin was washed with DMF, CH₂Cl₂, and MeOH the dried *in vacuo.*

### General protocol for resin cleavage

The dried resin was treated with TFA/Phenol/Thioanisole/1,2-Ethanedithiol/water (10mL/0.75g/0.5mL/0.25mL/0.5mL) and the mixture was shaken at room temperature for 3h. The filtrate was collected in a cold diethyl ether solution and the beads washed with TFA. The solution was centrifuged at 3000rpm for 2min. The precipitate was washed in a cold diethyl ether solution and centrifuged at 3000rpm for 2 min. The diethyl ether solution was eliminated and the precipitate was dried *in vacuo.* The crude product was purified by semi-preparative RP-HPLC and lyophilized.

### EXAMPLE 2: PEPTIDE ANALOGS SYNTHESIZED AND THEIR CHARACTERIZATION

### Apelin Analogs

### Synthesis of LE-122 and LE-124

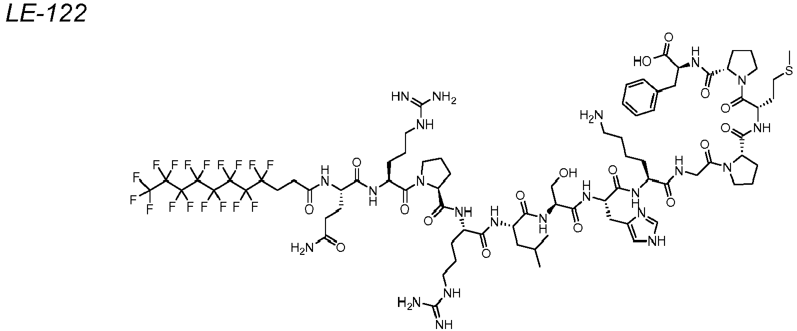

Fmoc-Gln-Arg-Pro-Arg-Leu-Ser-His-Lys-Gly-Pro-Met-Pro-Phe-Wang resin (16 µmol), 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general protocol, affording the title compound (18.9 mg, 47.7%) as a white solid. t_{R} =11.05 min (>95% purity at 220 nm); HRMS (ESI) calcd for C₈₀H₁₁₄F₁₇N₂₃O₁₇S: 2023.82123; found: 2023.82752. 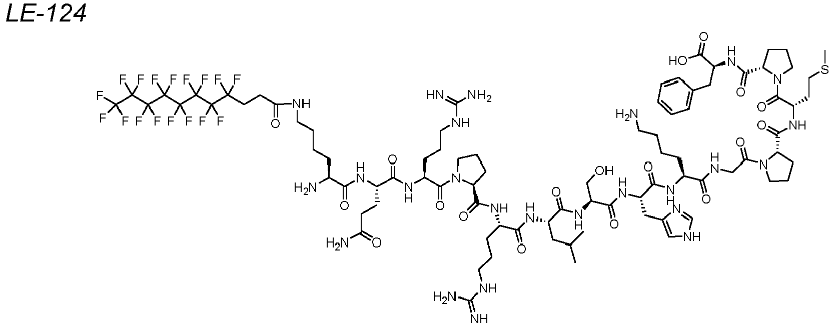 Fmoc-Gln-Arg-Pro-Arg-Leu-Ser-His-Lys-Gly-Pro-Met-Pro-Phe-Wang resin (16 µmol), Boc-Lys(Fmoc)-OH (4 equiv), HBTU (3.8 equiv), HOBt (4 equiv) and DIEA (12 equiv) were reacted according the general procedure. Then, 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general protocol, affording the title compound (14.5 mg, 35%) as a white solid. t_{R} =10.68 min (>95% purity at 220 nm); HRMS (ESI) calcd for C₈₆H₁₂₆F₁₇N₂₅O₁₈S: 2151.91619; found: 2151.91393.

### Characterization

### Affinity

Evaluation of the agonist activity of compounds at the human APJ receptor expressed in transfected CHO cells, determined by measuring their effects on cAMP modulation using the HTRF detection method. Experimental protocol: The cells are suspended in HBSS buffer (Invitrogen) complemented with 20 mM HEPES (pH 7.4) and 500 µM IBMX, then distributed in microplates at a density of 1.5x104 cells/well in the presence of either of the following: HBSS (basal control), the reference agonist at 30 nM (stimulated control) or various concentrations (EC50 determination), or the test compounds. Thereafter, the adenylyl cyclase activator NKH 477 is added at a final concentration of 0.3 µM. Following 10 min incubation at 37°C, the cells are lysed and the fluorescence acceptor (D2-labeled cAMP) and fluorescence donor (anti-cAMP antibody labeled with europium cryptate) are added. After 60 min at room temperature, the fluorescence transfer is measured at λex=337 nm and λem=620 and 665 nm using a microplate reader (Envison, Perkin Elmer). The cAMP concentration is determined by dividing the signal measured at 665 nm by that measured at 620 nm (ratio). The results are expressed as a percent of the control response to 30 nM apelin-13. The standard reference agonist is apelin-13, which is tested in each experiment at several concentrations to generate a concentration-response curve from which its EC50 value is calculated.

### IC50

Evaluation of the affinity of compounds for the human apelin receptor in transfected CHO cells determined in a radioligand binding assay. Experimental protocol : Cell membrane homogenates (1 µg protein) are incubated for 120 min at 22°C with 0.03 nM [125l](Glp65, Nle75, Tyr77)-apelin-13 in the absence or presence of the test compound in a buffer containing 50 mM Hepes/NaOH (pH 7.4), 100 mM NaCl, 10 mM KCl, 5 mM MgCl2, 1 mM EDTA, 0.04% bacitracin and 0.1% BSA. Nonspecific binding is determined in the presence of 1 µM apelin-13. Following incubation, the samples are filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters are dried then counted for radioactivity in a scintillation counter (Topcount, Packard) using a scintillation cocktail (Microscint 0, Packard). The results are expressed as a percent inhibition of the control radioligand specific binding. The standard reference compound is apelin-13, which is tested in each experiment at several concentrations to obtain a competition curve from which its IC50 is calculated.

### Solubility study

The solubility of each fluoropeptide was evaluated after dissolution in water to reach 100 µM. The resulting solution was vortexed 1 min following by 1 min in bath sonication. Solubility was then assessed by visual observation of the resulting dispersion (Clear/Cloudy and presence of particulates).

### Human plasma stability

This procedure is designed to determine the stability of a test compound in blood or plasma from human or animal species in a 96-well plate format. The test compound is quantified at 5 time points by HPLC-MS/MS analysis.
Test concentration : 1 µM with a final DMSO concentration of 0.5 %. Experimental protocol : Blood or plasma are pre-warmed at 37 °C water bath for 5 min, followed by addition of the test compound. The incubation is performed in a 37 °C water bath for 2 h. An aliquot of the incubation mixture is transferred to acetonitrile at 0, 0.5, 1, 1.5 and 2 h, respectively. Samples are then mixed and centrifuged. Supernatants are used for HPLC-MS/MS analysis. Reference compounds Propoxycaine and propantheline are tested simultaneously with the test compound in each assay. Analytical methods Samples are analyzed by HPLC-MS/MS using selected reaction monitoring. The HPLC system consists of a binary LC pump with autosampler, a C-18 column, and a gradient. Conditions may be adjusted as necessary. Data analysis Peak areas corresponding to the test compound are recorded. The compound remaining (%) is calculated by comparing the peak area at each time point to time zero. The half-life is calculated from the slope of the initial linear range of the logarithmic curve of compound remaining (%) vs. time, assuming first order kinetics

The results are presented in the table below:
human APJ (apelin) receptor

| Peptide | Affinity % of inhibition at 10 µM | IC50 cAMP (nM) | *Solubility* in water (µM) | Human plasma stability (t_{1/2}, min) |
|---|---|---|---|---|
| **pE13F** | **101.2** | **24** | **>100** | **5** |
| LE-122 | 100.0 | 2.7 | >100 | 1241 |
| LE-124 | 101.3 | 1.3 | - | 357 |

These results demonstrate that the incorporation of the fluorocarbon chain has no impact on both the binding affinity and the functional activity of the resulting peptides, regardless of the location of the fluorocarbon chain, either on the epsilon or on the alpha-amino group of the apelin peptide. Noteworthy, the solubility of both fluoropeptide is above 100 µM in water. Finally, the location of the chain has an impact on the metabolic stability of the fluoropeptide in human plasma. Indeed, the more stable construct is obtained when the fluorocarbon chain is introduced on the alpha-amino part of the peptide (LE-122, t_{1/2} > 1241 min).

### Angiotensin II analogs

### Synthesis of LE-120

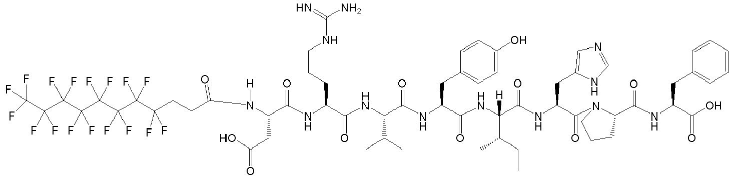

Fmoc-Asp-Arg-Val-Tyr-Ile-His-Pro-Phe-Wang resin (50 µmol), 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general protocol, affording the title compound (41.4 mg, 47%) as a white solid. t_{R} = 12.94 min (> 95% purity at 220 nm); HRMS (ESI) calcd for C₆₁H₇₄F₁₇N₁₃O₁₃: 15109.52576; found: 1519.52309.

### Characterization

### Affinity

Evaluation of the agonist activity of compounds at the human AT1 receptor expressed in transfected HEK-293 cells, determined by measuring their effect on cytosolic Ca2+ ion mobilization using a fluorimetric detection method. Experimental protocol: The cells are suspended in DMEM buffer (Invitrogen), then distributed in microplates at a density of 4.104 cells/well. The fluorescent probe (Fluo4 Direct, Invitrogen) mixed with probenicid in HBSS buffer (Invitrogen) complemented with 20 mM Hepes (Invitrogen) (pH 7.4) is then added into each well and equilibrated with the cells for 60 min at 37°C then 15 min at 22°C. Thereafter, the assay plates are positioned in a microplate reader (CellLux, PerkinElmer) which is used for the addition of the test compound, reference agonist or HBSS buffer (basal control), and the measurements of changes in fluorescence intensity which varies proportionally to the free cytosolic Ca2+ ion concentration. For stimulated control measurements, angiotensin-II at 30 nM is added in separate assay wells. The results are expressed as a percent of the control response to 30 nM angiotensin-II. The standard reference agonist is angiotensin-II, which is tested in each experiment at several concentrations to generate a concentration-response curve from which its EC50 value is calculated.

### IC50

Evaluation of the affinity of compounds for the human angiotensin-II AT1 receptor in transfected HEK-293 cells determined in a radioligand binding assay. Experimental protocol : Cell membrane homogenates (8 µg protein) are incubated for 120 min at 37°C with 0.05 nM [125l][Sar1-Ile8]angiotensin-II in the absence or presence of the test compound in a buffer containing 50 mM Tris-HCl (pH 7.4), 5 mM MgCl2, 1 mM EDTA and 0.1% BSA. Nonspecific binding is determined in the presence of 10 µM angiotensin II. Following incubation, the samples are filtered rapidly under vacuum through glass fiber filters (GF/B, Packard) presoaked with 0.3% PEI and rinsed several times with ice-cold 50 mM Tris-HCl using a 96-sample cell harvester (Unifilter, Packard). The filters are dried then counted for radioactivity in a scintillation counter (Topcount, Packard) using a scintillation cocktail (Microscint 0, Packard). The results are expressed as a percent inhibition of the control radioligand specific binding. The standard reference compound is saralasin, which is tested in each experiment at several concentrations to obtain a competition curve from which its IC50 is calculated.

### Solubility study

The solubility of the fluoropeptide was evaluated after dissolution in water to reach 100 µM. The resulting solution was vortexed 1 min following by 1 min in bath sonication. Solubility was then assessed by visual observation of the resulting dispersion (Clear/Cloudy and presence of particulates).

### Human plasma stability

This procedure is designed to determine the stability of a test compound in blood or plasma from human or animal species in a 96-well plate format. The test compound is quantified at 5 time points by HPLC-MS/MS analysis.
Test concentration : 1 µM with a final DMSO concentration of 0.5 %. Experimental protocol : Blood or plasma are pre-warmed at 37 °C water bath for 5 min, followed by addition of the test compound. The incubation is performed in a 37 °C water bath for 2 h. An aliquot of the incubation mixture is transferred to acetonitrile at 0, 0.5, 1, 1.5 and 2 h, respectively. Samples are then mixed and centrifuged. Supernatants are used for HPLC-MS/MS analysis. Reference compounds Propoxycaine and propantheline are tested simultaneously with the test compound in each assay. Analytical methods Samples are analyzed by HPLC-MS/MS using selected reaction monitoring. The HPLC system consists of a binary LC pump with autosampler, a C-18 column, and a gradient. Conditions may be adjusted as necessary. Data analysis Peak areas corresponding to the test compound are recorded. The compound remaining (%) is calculated by comparing the peak area at each time point to time zero. The half-life is calculated from the slope of the initial linear range of the logarithmic curve of compound remaining (%) vs. time, assuming first order kinetics

The results are presented in the table below :

| human AT1R | | | | |
|---|---|---|---|---|
| Peptide | Affinity % of inhibition at 10 µM | EC50 Ca²⁺ production (nM) | Solubility in water (µM) | Human plasma stability (t_{1/2}, min) |
| **Angiotensin II** | **100.2** | **14** | **>100** | **85** |
| LE120 | 66.4 | 460 | >100 | >1440 |

These results indicate that the incorporation of the fluorocarbon chain has an impact on both the affinity and the functional activity compared to the native peptide. Indeed, the affinity of LE120 is significantly decreased altogether with the efficacy from 14 vs 460 nM, respectively. However, as previously described for apelin-13, the human plasma stability is greatly improved from 85 min for the native peptide to >1440 min for the fluoropeptide, again demonstrating the beneficial effect of the fluorocarbon chain on the metabolic stability.

### Oxytocin analogs

### Synthesis of SR-11-74

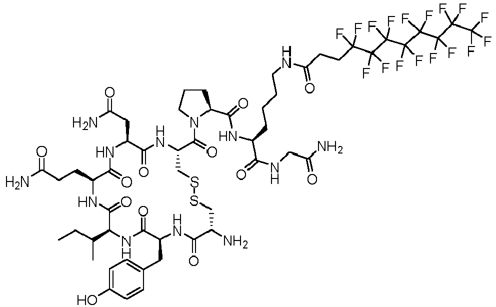

Fmoc-Cys-Tyr-Ile-Gln-Asn-Cys-Pro-Lys-Gly-NH₂-Rink resin (165 µmol), 4,4,5,5,6,6,7,7,8,8,9,9,10,10,11,11-heptadecafluoroundecanoic acid (2 equiv), HBTU (2 equiv), HOBt (1.9 equiv) and DIEA (8 equiv) were reacted according the general protocol, affording the title compound (21.1 mg, 8%) as a white solid. t_{R} =12.78 min (>95% purity at 220,8 nm); HRMS (ESI) calcd for C₅₄H₇₀F₁₇N₁₃O₁₃S₂: 1495.4386; found: 1495.43437.

### Characterization

### Affinity

Evaluation of the agonist activity of compounds at the human OT receptor endogenously expressed in ECV304 cells, determined by measuring their effect on cytosolic Ca2+ ion mobilization using a fluorimetric detection method. Experimental protocol : The cells are suspended in DMEM buffer (Invitrogen), then distributed in microplates at a density of 3.104 cells/well. The fluorescent probe (Fluo4 Direct, Invitrogen) mixed with probenicid in HBSS buffer (Invitrogen) complemented with 20 mM Hepes (Invitrogen) (pH 7.4) is then added into each well and equilibrated with the cells for 60 min at 37°C then 15 min at 22°C. Thereafter, the assay plates are positioned in a microplate reader (CellLux, PerkinElmer) which is used for the addition of the test compound, reference agonist or HBSS buffer (basal control), and the measurements of changes in fluorescence intensity which varies proportionally to the free cytosolic Ca2+ ion concentration. For stimulated control measurements, oxytocin at 3 µM is added in separate assay wells. The results are expressed as a percent of the control response to 3 µM oxytocin. The standard reference agonist is oxytocin, which is tested in each experiment at several concentrations to generate a concentration-response curve from which its EC50 value is calculated.

### IC50

Evaluation of the affinity of compounds for the human vasopressin Oxytocin receptor in transfected Chem-1 cells determined in a radioligand binding assay. Experimental protocol : Cell membrane homogenates (about 10 µg protein) are incubated for 120 min at 22°C with 0.8 nM [3H]Oxytocin in the absence or presence of the test compound in a buffer containing 50 mM Tris-HCl (pH 7.4), 5 mM MgCl2 and 0.1% BSA. Nonspecific binding is determined in the presence of 1 µM Oxytocin. Following incubation, the samples are filtered rapidly under vacuum through glass fiber filters (GF/B, Packard) presoaked with 0.3% PEI and rinsed several times with ice-cold 50 mM Tris-HCl and 0.1% BSA using a 96-sample cell harvester (Unifilter, Packard). The filters are dried then counted for radioactivity in a scintillation counter (Topcount, Packard) using a scintillation cocktail (Microscint 0, Packard). The results are expressed as a percent inhibition of the control radioligand specific binding. The standard reference compound is Oxytocin, which is tested in each experiment at several concentrations to obtain a competition curve from which its IC50 is calculated.

### Solubility study

The solubility of the fluoropeptide was evaluated after dissolution in water to reach 100 µM. The resulting solution was vortexed 1 min following by 1 min in bath sonication. Solubility was then assessed by visual observation of the resulting dispersion (Clear/Cloudy and presence of particulates).

### Human plasma stability

This procedure is designed to determine the stability of a test compound in blood or plasma from human or animal species in a 96-well plate format. The test compound is quantified at 5 time points by HPLC-MS/MS analysis.
Test concentration : 1 µM with a final DMSO concentration of 0.5 %. Experimental protocol : Blood or plasma are pre-warmed at 37 °C water bath for 5 min, followed by addition of the test compound. The incubation is performed in a 37 °C water bath for 2 h. An aliquot of the incubation mixture is transferred to acetonitrile at 0, 0.5, 1, 1.5 and 2 h, respectively. Samples are then mixed and centrifuged. Supernatants are used for HPLC-MS/MS analysis. Reference compounds Propoxycaine and propantheline are tested simultaneously with the test compound in each assay. Analytical methods Samples are analyzed by HPLC-MS/MS using selected reaction monitoring. The HPLC system consists of a binary LC pump with autosampler, a C-18 column, and a gradient. Conditions may be adjusted as necessary. Data analysis Peak areas corresponding to the test compound are recorded. The compound remaining (%) is calculated by comparing the peak area at each time point to time zero. The half-life is calculated from the slope of the initial linear range of the logarithmic curve of compound remaining (%) vs. time, assuming first order kinetics.

The results are presented in the table below :

| human oxytocin receptor | | | | |
|---|---|---|---|---|
| Peptide | Affinity % of inhibition à 10 nM | EC50 Ca²⁺ production (nM) | Solubility in water (µM) | Human plasma stability (t_{1/2}, min) |
| **Oxytocin** | **102.2** | **42** | **>100** | **147** |
| SR-11-74 | 91.3 | <25% at 100 nM | >100 | 239 |

In the case of cyclic oxytocin, the incorporation of the fluorocarbon chain has a profound effect on the functional activity of the peptide (EC50 from 42 nM for the native peptide to >25% at 100 nM for the fluoroocytocin). In another hand, the presence of the fluorocarbon chain has a low impact on the human plasma stability (t_{1/2} = 147 min for the oxytocin *vs* 239 min for the fluoro-oxytocin).

### Reference listing

1. Fosgerau, K.; Hoffmann, T. Peptide therapeutics: current status and future directions. Drug Discovery Today 2015, 20, 122-128
2. Vlieghe, P.; Lisowski, V.; Martinez, J.; Khrestchatisky, M. Synthetic therapeutic peptides: science and market. Drug Discovery Today 2010, 15, 40-56
3. Congreve, M.; Langmead, C. J.; Mason, J. S.; Marshall, F. H. Progress in Structure Based Drug Design for G Protein-Coupled Receptors. J. Med. Chem. 2011, 54, 4283-4311
4. Narayanan S, Harris DL, Maitra R, Runyon SP. Regulation of the Apelinergic System and Its Potential in Cardiovascular Disease: Peptides and Small Molecules as Tools for Discovery. J Med Chem. 2015 Oct 22;58(20):7913-27
5. Marc Y, Llorens-Cortes C. The role of the brain renin-angiotensin system in hypertension: implications for new treatment.. Prog Neurobiol. 2011 Oct;95(2):89-103
6. Modi, M. E.; Young, L. J. The oxytocin system in drug discovery for autism: Animal models and novel therapeutic strategies. Horm. Behav. 2012, 61, 340-350

## Claims

1. A metabolically stable and non-immunogenic peptide analog completely hydrosoluble at physiological pH having a peptide covalently linked to a fluorocarbon group, directly or through a linker selected from the group consisting of a PEG or a peptide having from 1 to 6 amino acids, either on the alpha-amino or the epsilon-amino group of at least one lysine of said peptide, when the linker is a lysine, the fluorocarbon group is directly linked to the epsilon-amino group of said linker;
but excluding an apelin analog having the following peptide of formula (I):
Lys-Phe-Xaa1-Arg-Xaa2-Arg-Pro-Arg-Xaa3-Ser-Xaa4-Lys-Xaa5-Pro-Xaa6-Pro-Xaa7 (I)
wherein said peptide of formula (I) is linked to a fluorocarbon group, an acetyl group, or an acyl group -C(O)R where R is a C₇₋₃₀ alkyl, directly or through a linker selected from the group consisting of PEG, lysine and arginine, either on the alpha-amino or the epsilon-amino group of at least one lysine of the peptide formula (I), and when the linker is a lysine, the fluorocarbon group, acetyl or acyl group is directly linked either on the epsilon-amino group of said linker and wherein :
Xaa1 is arginine (R) or D-isomer arginine (R_{D});
Xaa2 is glutamine (Q) or D-isomer glutamine (Q_{D})
Xaa3 is leucine (L) or D-isomer leucine (L_{D});
Xaa4 is histidine (H) or α-aminoisobutyric acid (Aib);
Xaa5 is alanine (A) or D-isomer alanine (A_{D}) or glycine (G);
Xaa6 is methionine (M) or norleucine (Nle);
Xaa7 is phenylalanine (F) of 4-Br phenylalanine (4-BrF).

2. The peptide analog according to claim 1, wherein said peptide is further covalently linked to an acetyl group and/or an acyl group -C(O)R where R is a C₇₋₃₀ alkyl.

3. The peptide analog according to either one of claim 1 or 2, wherein said peptide covalently linked to the fluorocarbon group is no more than 13 amino acid residues.

4. The peptide analog according to any one of claim 1 to 3, wherein said fluorocarbon group linked to said peptide has the following formula (II):
CₘFₙ-C_{y}Hₓ(L) (II)
wherein m=3 to 30, n≤2m+1, y=0 to 2, x≤2y, (m+y)=3 to 30, and L, which is optional, is a linker selected from the group consisting of a PEG or a peptide having from 1 to 6 amino acids.

5. The peptide analog according to any one of claims 1 to 4, wherein said acyl group has the following formula (III):
CH₃-C_{y}Hₓ-C(O)- (III)
wherein y=7 to 30, x=2y.

6. The peptide analog according to any one of claims 1 to 5, which is selected from:
i) an apelin analog having said peptide of the formula (IV):
Xaa1-Arg-Pro-Xaa2-Leu-Xaa3-Xaa4-Xaa5-Gly-Pro-Xaa6-Pro-Xaa7 (IV)
and wherein
Xaa1 is L- or D-glutamine (QL or QD) or alanine (A);
Xaa2 is arginine (R) or lysine (K) or D-norleucine (Nle);
Xaa3 is serine (S) or alanine (A);
Xaa4 is histidine (H) or alanine (A);
Xaa5 is lysine (K) or norleucine (Nle);
Xaa6 is methionine (M), leucine (L), phenylalanine (F) or norleucine (Nle);
Xaa7 is phenylalanine (F), 4-Br phenylalanine (4-BrF), 4-(Obenzyl)phenylalanine (4-ObnF) or p-benzoyl phenylalanine (Bpa); and
ii) an apelin analog with an amino acid sequence having at least 80% identity with the sequence of (i).

7. A peptide analog according to any one of claims 1 to 5, which is selected from the group consisting of:
i) an angiotensin II analog having said peptide of the formula (V):
Xaa1-Arg-Val-Tyr-Ile-His-Pro-Xaa2 (V)
and wherein
Xaa1 is aspartic acid (D) or sarcosine;
Xaa2 is phenylalanine or OH; and
ii) an angiotensin II analog with an amino acid sequence having at least 80% identity with the sequence of (i).

8. A peptide analog according to any one of claims 1 to 5, which is selected from the group consisting of:
i) an oxytocin analog having said peptide of the formula (VI):
Cys-Tyr-Ile-Xaa1-Asp-Cys-Xaa2-Xaa3-Gly (VI)
and wherein
Xaa1 is glutamine or threonine;
Xaa2 is proline or glycine;
Xaa3 is leucine, lysine or proline;
ii) an oxytocin analog with an amino acid sequence having at least 80% identity with the sequence of (i).

9. The peptide analog according to any one of claims 1 to 8, for use as a drug.

10. The peptide analog according to any one of claims 1 to 8, for use in the treatment of a GPCR-related disease selected from:
- cardiovascular disease: heart failure, kidney diseases (e.g. renal failure, nephritis, etc...), hypertension, pulmonary hypertension, cirrhosis, atherosclerosis, pulmonary emphysema, pulmonary oedema, stroke, brain ischemia, myocardial impairment in sepsis;
- the syndrome of inappropriate antidiuretic hormone (SIADH);
- metabolic diseases: obesity, anorexia, hyperphagia, polyphagia, hypercholesterolemia, hyperglyceridemia, hyperlipemia;
- various types of dementia: senile dementia, cerebrovascular dementia, dementia due to genealogical denaturation degeneratie disesases, dementia resulting from infectious diseases, deentia associated with endocrine diseases, metabolic diseases, or poisoning, dementia caused by tumors, and dementia due to traumatic diseases, depression, hyperactive child syndrome, disturbance of consciousness, anxiety disorder, schizophrenia, phobia;
- pain and hyperalgesia.

11. A pharmaceutical composition comprising a peptide analog according to any one of claims 1 to 8, and one or more pharmaceutically acceptable excipient.


SEQUENCE LISTING

<110> UNIVERSITE DE STRASBOURG
INSERM
CNRS
BONNET, Dominique
LLORENS CORTES, Catherine
ITURRIOZ, Xavier

<120> METABOLICALLY STABLE PEPTIDE ANALOGS

<130> BNT220197EP00

<160> 122

<170> PatentIn version 3.5

<210> 1
<211> 17
<212> PRT
<213> Artificial Sequence

<220>
<223> apelin analog (formula I)


<220>
<221> MISC_FEATURE
<222> (3)..(3)
<223> Xaa is arginine or D-isomer arginine

<220>
<221> MISC_FEATURE
<222> (5)..(5)
<223> Xaa is glutamine or D-isomer glutamine

<220>
<221> MISC_FEATURE
<222> (9)..(9)
<223> Xaa is leucine or D-isomer leucine

<220>
<221> misc_feature
<222> (11)..(11)
<223> Xaa can be any naturally occurring amino acid

<220>
<221> MISC_FEATURE
<222> (13)..(13)
<223> Xaa is alanine or D-isomer alanine or glycine

<220>
<221> MISC_FEATURE
<222> (15)..(15)
<223> Xaa is methionine or norleucine

<220>
<221> MISC_FEATURE
<222> (17)..(17)
<223> Xaa is phenylalanine or 4-Br-phenylalanine

<400> 1

Lys Phe Xaa Arg Xaa Arg Pro Arg Xaa Ser Xaa Lys Xaa Pro Xaa Pro
1               5                   10                  15

Xaa

<210>  2
<211>  13
<212>  PRT
<213>  Artificial Sequence

<220>
<223>  apelin analog (formula IV)

<220>
<221>  MISC_FEATURE
<222>  (1)..(1)
<223>  Xaa is L- or D-glutamine or alanine

<220>
<221>  MISC_FEATURE
<222>  (4)..(4)
<223>  Xaa is arginine or lysine or D-norleucine

<220>
<221>  MISC_FEATURE
<222>  (6)..(6)
<223>  Xaa is serine or alanine

<220>
<221>  MISC_FEATURE
<222>  (7)..(7)
<223>  Xaa is histidine or alanine

<220>
<221>  MISC_FEATURE
<222>  (8)..(8)
<223>  Xaa is lysine or norleucine

<220>
<221>  MISC_FEATURE
<222>  (11)..(11)
<223>  Xaa is methionine, leucine, phenylalanine or norleucine

<220>
<221>  MISC_FEATURE
<222>  (13)..(13)
<223>  Xaa is phenylalanine, 4-Br-phenylalanine,
       4-(Obenzyl)phenylalanine or p-benzoyl-phenylalanine

<400>  2

Xaa Arg Pro Xaa Leu Xaa Xaa Xaa Gly Pro Xaa Pro Xaa
1               5                   10

<210>  3
<211>  8
<212>  PRT
<213>  Artificial Sequence

<220>
<223>  angiotensin II analog


<220>
<221>  MISC_FEATURE
<222>  (1)..(1)
<223>  Xaa is aspartic acid or sarcosine

<220>
<221>  MISC_FEATURE
<222>  (8)..(8)
<223>  Xaa is phenylalanine or OH

<400>  3

Xaa Arg Val Tyr Ile His Pro Xaa
1               5


<210>  4
<211>  9
<212>  PRT
<213>  Artificial Sequence

<220>
<223>  oxytocin analog


<220>
<221>  MISC_FEATURE
<222>  (4)..(4)
<223>  Xaa is glutamine or threonine

<220>
<221>  MISC_FEATURE
<222>  (7)..(7)
<223>  Xaa is proline or glycine

<220>
<221>  MISC_FEATURE
<222>  (8)..(8)
<223>  Xaa is leucine, lysine or proline

<400>  4

Cys Tyr Ile Xaa Asp Cys Xaa Xaa Gly
1               5


<210>  5
<211>  15
<212>  PRT
<213>  Artificial Sequence

<220>
<223>  Balixafortide

<400>  5

Ala Cys Ser Ala Pro Arg Tyr Cys Tyr Gln Lys Pro Pro Tyr His
1               5                   10                  15

<210>  6
<211>  29
<212>  PRT
<213>  Artificial Sequence

<220>
<223>  BIS-5409 / MR-409

<400>  6

Tyr Ala Asp Ala Ile Phe Thr Asn Ser Tyr Arg Lys Val Leu Gly Gln
1               5                   10                  15

Leu Ser Ala Arg Lys Leu Leu Gln Asp Ile Met Ser Arg
            20                  25

<210>  7
<211>  20
<212>  PRT
<213>  Artificial Sequence

<220>
<223>  Bivalirudin

<400>  7

Phe Pro Arg Pro Gly Gly Gly Gly Asn Gly Asp Phe Glu Glu Ile Pro
1               5                   10                  15

Glu Glu Tyr Leu
            20

<210>  8
<211>  5
<212>  PRT
<213>  Artificial Sequence

<220>
<223>  BQ-123

<400>  8

Asp Pro Val Leu Tyr
1               5

<210>  9
<211>  28
<212>  PRT
<213>  Artificial Sequence

<220>
<223>  Carperitide

<400>  9

Ser Leu Arg Arg Ser Ser Cys Phe Gly Gly Arg Met Asp Arg Ile Gly

1               5                   10                  15

Ala Gln Ser Gly Leu Gly Cys Asn Ser Phe Arg Tyr
            20                  25

<210>  10
<211>  18
<212>  PRT
<213>  Artificial Sequence

<220>
<223>  CEL-1000

<400>  10

Asp Gly Gln Glu Glu Lys Ala Gly Val Val Ser Thr Gly Leu Ile Gly
1               5                   10                  15

Gly Gly

<210>  11
<211>  37
<212>  PRT
<213>  Artificial Sequence

<220>
<223>  Cenderitide

<400>  11

Gly Leu Ser Lys Gly Cys Phe Gly Leu Lys Leu Asp Arg Ile Gly Ser
1               5                   10                  15

Met Ser Gly Leu Gly Cys Pro Ser Leu Arg Asp Pro Arg Pro Asn Ala
            20                  25                  30

Pro Ser Thr Ser Ala
        35

<210>  12
<211>  23
<212>  PRT
<213>  Artificial Sequence

<220>
<223>  CGEN-856S

<400>  12

Phe Leu Gly Tyr Cys Ile Tyr Leu Asn Lys Arg Arg Gly Asp Pro Ala
1               5                   10                  15

Phe Lys Arg Arg Leu Arg Asp
            20

<210>  13
<211>  10
<212>  PRT
<213>  Artificial Sequence

<220>
<223>  Des-Asp Angiotensin 1

<400>  13

Asp Arg Val Tyr Ile His Pro Phe His Leu
1               5                   10


<210>  14
<211>  65
<212>  PRT
<213>  Artificial Sequence

<220>
<223>  Desirudin

<400>  14

Val Val Tyr Thr Asp Cys Thr Glu Ser Gly Gln Asn Leu Cys Leu Cys
1               5                   10                  15


Glu Gly Ser Asn Val Cys Gly Gln Gly Asn Lys Cys Ile Leu Gly Ser
            20                  25                  30


Asp Gly Glu Lys Asn Gln Cys Val Thr Gly Glu Gly Thr Pro Lys Pro
        35                  40                  45


Gln Ser His Asn Asp Gly Asp Phe Glu Glu Ile Pro Glu Glu Tyr Leu
    50                  55                  60


Gln
65


<210>  15
<211>  4
<212>  PRT
<213>  Artificial Sequence

<220>
<223>  Elamipretide


<220>
<221>  MISC_FEATURE
<222>  (2)..(2)
<223>  Xaa is 2,6-dimethyltyrosyl

<400>  15

Arg Xaa Lys Phe

1



<210>  16
<211>  28
<212>  PRT
<213>  Artificial Sequence

<220>
<223>  FX-06

<400>  16

Arg Gly His Arg Pro Leu Asp Lys Lys Arg Glu Glu Ala Pro Ser Leu
1               5                   10                  15


Arg Pro Ala Pro Pro Pro Ile Ser Gly Gly Gly Tyr
            20                  25



<210>  17
<211>  8
<212>  PRT
<213>  Artificial Sequence

<220>
<223>  LJPC-501 (Angiotensin II)

<400>  17

Asp Arg Val Tyr Ile His Pro Phe
1               5



<210>  18
<211>  37
<212>  PRT
<213>  Artificial Sequence

<220>
<223>  MP-3167 (Vastiras)

<400>  18

Ala Val Ser Asn Ala Asp Leu Met Asp Phe Lys Asn Leu Leu Asp His
1               5                   10                  15


Leu Glu Glu Lys Met Pro Leu Glu Asp Glu Val Val Pro Pro Gln Val
            20                  25                  30


Leu Ser Glu Arg Asn
        35



<210>  19
<211>  19
<212>  PRT
<213>  Artificial Sequence

<220>

<223>  NA-1

<400>  19

Tyr Arg Lys Lys Arg Arg Gln Arg Arg Arg Lys Leu Ser Ser Ile Glu
1               5                   10                  15

Ser Asp Val

<210>  20
<211>  40
<212>  PRT
<213>  Artificial Sequence

<220>
<223>  NBI-69734 (Urocortin II)

<400>  20

Ile Val Leu Ser Leu Asp Val Pro Ile Gly Leu Leu Gln Ile Leu Leu
1               5                   10                  15

Glu Gln Ala Arg Ala Arg Ala Ala Arg Glu Gln Ala Thr Thr Asn Ala
            20                  25                  30

Arg Ile Leu Ala Arg Val Gly His
        35                  40

<210>  21
<211>  32
<212>  PRT
<213>  Artificial Sequence

<220>
<223>  Nesiritide

<400>  21

Ser Pro Lys Met Val Gln Gly Ser Gly Cys Phe Gly Arg Lys Met Asp
1               5                   10                  15

Arg Ile Ser Ser Ser Ser Gly Leu Gly Cys Lys Val Leu Arg Arg His
            20                  25                  30

<210>  22
<211>  14
<212>  PRT
<213>  Artificial Sequence

<220>
<223>  PL-3994

<220>
<221>  MISC_FEATURE

<222>  (7)..(7)
<223>  Xaa is Nle

<400>  22

Cys His Phe Ala Gly Arg Xaa Asp Arg Ile Ser Cys Tyr Arg
1               5                   10

<210>  23
<211>  44
<212>  PRT
<213>  Artificial Sequence

<220>
<223>  RGN-352 (Thymosin beta-4)

<400>  23

Met Ser Asp Lys Pro Asp Met Ala Glu Ile Glu Lys Phe Asp Lys Ser
1               5                   10                  15

Lys Leu Lys Lys Thr Glu Thr Gln Glu Lys Asn Pro Leu Pro Ser Lys
            20                  25                  30

Glu Thr Ile Glu Gln Glu Lys Gln Ala Gly Glu Ser
        35                  40

<210>  24
<211>  17
<212>  PRT
<213>  Artificial Sequence

<220>
<223>  Solnatide

<400>  24

Cys Gly Gln Arg Glu Thr Pro Glu Gly Ala Glu Ala Lys Pro Trp Tyr
1               5                   10                  15

Cys

<210>  25
<211>  8
<212>  PRT
<213>  Artificial Sequence

<220>
<223>  TRV027

<400>  25

Gly Arg Val Tyr Ile His Pro Ala
1               5

<210>  26
<211>  32
<212>  PRT
<213>  Artificial Sequence

<220>
<223>  Ularitide (Urodilatin)

<400>  26

Thr Ala Pro Arg Ser Leu Arg Arg Ser Ser Cys Phe Gly Gly Arg Met
1               5                   10                  15

Asp Arg Ile Gly Ala Gln Ser Gly Leu Gly Cys Asn Ser Phe Arg Tyr
            20                  25                  30

<210>  27
<211>  17
<212>  PRT
<213>  Artificial Sequence

<220>
<223>  acALY-18

<400>  27

Ala Leu Tyr Asp Lys Gly Tyr Thr Ser Lys Glu Gln Lys Asp Cys Val
1               5                   10                  15

Gly

<210>  28
<211>  12
<212>  PRT
<213>  Artificial Sequence

<220>
<223>  Cibinetide

<220>
<221>  MISC_FEATURE
<222>  (1)..(1)
<223>  Xaa is 5-Oxo-

<400>  28

Xaa Pro Glu Gln Leu Glu Arg Ala Leu Asp Ser Ser
1               5                   10

<210>  29
<211>  17
<212>  PRT
<213>  Artificial Sequence

<220>
<223>  COG-112

<400>  29

Arg Gln Ile Lys Ile Trp Phe Gln Asn Arg Arg Met Lys Trp Lys Lys
1               5                   10                  15

Cys

<210>  30
<211>  39
<212>  PRT
<213>  Artificial Sequence

<220>
<223>  corticotropin

<400>  30

Ser Tyr Ser Met Glu His Phe Arg Trp Gly Lys Pro Val Gly Lys Lys
1               5                   10                  15

Arg Arg Pro Val Lys Val Tyr Pro Asp Gly Ala Glu Asp Gln Leu Ala
            20                  25                  30

Glu Ala Phe Pro Leu Glu Phe
        35

<210>  31
<211>  5
<212>  PRT
<213>  Artificial Sequence

<220>
<223>  PMZ-2123

<400>  31

Asp Pro Val Leu Trp
1               5

<210>  32
<211>  40
<212>  PRT
<213>  Artificial Sequence

<220>
<223>  PT-320

<400>  32

Ser His Gly Glu Gly Thr Phe Ala Thr Ser Asp Leu Ser Lys Gln Met
1               5                   10                  15

Gln Gln Gln Ala Val Arg Leu Phe Ile Gln Trp Leu Lys Asp Gly Gly
            20                  25                  30

Pro Ser Ser Gly Ala Pro Pro Pro
        35                  40

<210>  33
<211>  25
<212>  PRT
<213>  Artificial Sequence

<220>
<223>  Ziconotide

<400>  33

Cys Lys Gly Lys Gly Ala Lys Cys Ser Arg Leu Met Tyr Asp Cys Cys
1               5                   10                  15

Thr Gly Ser Cys Arg Ser Gly Lys Cys
            20                  25

<210>  34
<211>  8
<212>  PRT
<213>  Artificial Sequence

<220>
<223>  AB-103

<400>  34

Ser Pro Met Leu Val Ala Tyr Asp
1               5

<210>  35
<211>  30
<212>  PRT
<213>  Artificial Sequence

<220>
<223>  AG-30/5C

<400>  35

Met Leu Lys Leu Ile Phe Leu His Arg Leu Lys Arg Met Arg Lys Arg
1               5                   10                  15

Leu Lys Arg Lys Leu Arg Leu Trp His Arg Lys Arg Tyr Lys
            20                  25                  30

<210>  36
<211>  14
<212>  PRT
<213>  Artificial Sequence

<220>
<223>  FOL-005

<400>  36

Val Asp Thr Tyr Asp Gly Asp Ile Ser Trp Tyr Gly Leu Arg
1               5                   10

<210>  37
<211>  25
<212>  PRT
<213>  Artificial Sequence

<220>
<223>  Granexin

<400>  37

Arg Gln Pro Lys Ile Trp Phe Pro Asn Arg Arg Lys Pro Trp Lys Lys
1               5                   10                  15

Arg Pro Arg Pro Asp Asp Leu Glu Ile
            20                  25

<210>  38
<211>  37
<212>  PRT
<213>  Artificial Sequence

<220>
<223>  LL-37

<400>  38

Leu Leu Gly Asp Phe Phe Arg Lys Ser Lys Glu Lys Ile Gly Lys Glu
1               5                   10                  15

Phe Lys Arg Ile Val Gln Arg Ile Lys Asp Phe Leu Arg Asn Leu Val
            20                  25                  30

Pro Arg Thr Glu Ser
        35

<210>  39
<211>  12
<212>  PRT
<213>  Artificial Sequence

<220>
<223>  Omiganan

<400>  39

Ile Leu Arg Trp Pro Trp Trp Pro Trp Arg Arg Lys
1               5                   10

<210>  40
<211>  25

<212>  PRT
<213>  Artificial Sequence

<220>
<223>  PXL-01

<400>  40

Glu Ala Thr Lys Cys Phe Gln Trp Gln Arg Asn Met Arg Lys Val Arg
1               5                   10                  15

Gly Pro Pro Val Ser Cys Ile Lys Arg
            20                  25

<210>  41
<211>  44
<212>  PRT
<213>  Artificial Sequence

<220>
<223>  RGN-137

<400>  41

Met Ser Asp Lys Pro Asp Met Ala Glu Ile Glu Lys Phe Asp Lys Ser
1               5                   10                  15

Lys Leu Lys Lys Thr Glu Thr Gln Glu Lys Asn Pro Leu Pro Ser Lys
            20                  25                  30

Glu Thr Ile Glu Gln Glu Lys Gln Ala Gly Glu Ser
        35                  40

<210>  42
<211>  20
<212>  PRT
<213>  Artificial Sequence

<220>
<223>  SR-0379

<400>  42

Met Leu Lys Leu Ile Phe Leu His Arg Leu Lys Arg Met Arg Lys Arg
1               5                   10                  15

Leu Lys Arg Lys
            20

<210>  43
<211>  18
<212>  PRT
<213>  Artificial Sequence

<220>
<223>  B-2088

<400>  43

Arg Gly Arg Lys Val Val Arg Arg Lys Lys Arg Arg Val Val Lys Arg
1               5                   10                  15

Gly Arg

<210>  44
<211>  31
<212>  PRT
<213>  Artificial Sequence

<220>
<223>  XG-102

<400>  44

Asp Gln Ser Arg Pro Val Gln Pro Phe Leu Asn Leu Thr Thr Pro Arg
1               5                   10                  15

Lys Pro Arg Pro Pro Arg Arg Arg Gln Arg Arg Lys Lys Arg Gly
            20                  25                  30

<210>  45
<211>  24
<212>  PRT
<213>  Artificial Sequence

<220>
<223>  Cosyntropin ER

<400>  45

Ser Tyr Ser Met Glu His Phe Arg Trp Gly Lys Pro Val Gly Lys Lys
1               5                   10                  15

Arg Arg Pro Val Lys Val Tyr Pro
            20

<210>  46
<211>  34
<212>  PRT
<213>  Artificial Sequence

<220>
<223>  FE-203799

<400>  46

His Gly Asp Gly Ser Phe Ser Asp Glu Met Phe Thr Ile Leu Asp Leu
1               5                   10                  15

Leu Ala Ala Arg Asp Phe Phe Ile Asn Trp Leu Ile Gln Thr Lys Ile
            20                  25                  30

Thr Asp

<210> 47
<211> 14
<212> PRT
<213> Artificial Sequence

<220>
<223> Linaclotide

<400> 47

Cys Cys Glu Tyr Cys Cys Asn Pro Ala Cys Thr Gly Cys Tyr
1               5                   10

<210> 48
<211> 8
<212> PRT
<213> Artificial Sequence

<220>
<223> Octreotide

<220>
<221> MISC_FEATURE
<222> (6)..(6)
<223> Xaa is DL-threonine

<220>
<221> MISC_FEATURE
<222> (8)..(8)
<223> Xaa is DL-threonine

<400> 48

Phe Cys Phe Trp Lys Xaa Cys Xaa
1               5

<210> 49
<211> 8
<212> PRT
<213> Artificial Sequence

<220>
<223> Octreotide Acetate

<400> 49

Phe Cys Phe Trp Lys Thr Cys Thr
1               5

<210> 50
<211> 16
<212> PRT
<213> Artificial Sequence

<220>
<223>  Plecanatide

<400>  50

Asn Asp Glu Cys Glu Leu Cys Val Asn Val Ala Cys Thr Gly Cys Leu
1               5                   10                  15


<210>  51
<211>  13
<212>  PRT
<213>  Artificial Sequence

<220>
<223>  Recanaclotide


<220>
<221>  MISC_FEATURE
<222>  (3)..(3)
<223>  Xaa is Ser(PO3H2)

<400>  51

Cys Cys Xaa Leu Cys Cys Asn Pro Ala Cys Thr Gly Cys
1               5                   10


<210>  52
<211>  14
<212>  PRT
<213>  Artificial Sequence

<220>
<223>  Somatostatin


<220>
<221>  MISC_FEATURE
<222>  (10)..(10)
<223>  Xaa is DL-threonine

<220>
<221>  MISC_FEATURE
<222>  (12)..(12)
<223>  Xaa is DL-threonine

<400>  52

Ala Gly Cys Lys Asn Phe Phe Trp Lys Xaa Phe Xaa Ser Cys
1               5                   10


<210>  53
<211>  33
<212>  PRT
<213>  Artificial Sequence

<220>
<223>  Teduglutide

<400>  53

His Gly Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  54
<211>  33
<212>  PRT
<213>  Artificial Sequence

<220>
<223>  ZP-1848

<400>  54

His Gly Glu Gly Thr Phe Ser Ser Glu Leu Ala Thr Ile Leu Asp Ala
1               5                   10                  15

Leu Ala Ala Arg Asp Phe Ile Ala Trp Leu Ile Ala Thr Lys Ile Thr
            20                  25                  30

Asp

<210>  55
<211>  13
<212>  PRT
<213>  Artificial Sequence

<220>
<223>  Afamelanotide

<220>
<221>  MISC_FEATURE
<222>  (4)..(4)
<223>  Xaa is Nle

<400>  55

Ser Tyr Ser Xaa Glu His Phe Arg Trp Gly Lys Pro Val
1               5                   10

<210>  56
<211>  8
<212>  PRT
<213>  Artificial Sequence

<220>
<223>  AZP-531

<400>  56

Ser Pro Glu His Gln Arg Val Gln
1               5


<210>  57
<211>  25
<212>  PRT
<213>  Artificial Sequence

<220>
<223>  LJPC-401

<400>  57

Asp Thr His Phe Pro Ile Cys Ile Phe Cys Cys Gly Cys Cys His Arg
1               5                   10                  15


Ser Lys Cys Gly Met Cys Cys Lys Thr
            20                  25


<210>  58
<211>  8
<212>  PRT
<213>  Artificial Sequence

<220>
<223>  Setmelanotide

<400>  58

Arg Cys Ala His Phe Arg Trp Cys
1               5


<210>  59
<211>  7
<212>  PRT
<213>  Artificial Sequence

<220>
<223>  TXA-127

<400>  59

Asp Arg Val Tyr Ile His Pro
1               5


<210>  60
<211>  13
<212>  PRT
<213>  Artificial Sequence

<220>
<223>  AMY-101


<220>

<221>  MISC_FEATURE
<222>  (9)..(9)
<223>  Xaa is Sarcosine

<400>  60

Tyr Ile Cys Val Trp Gln Asp Trp Xaa His Arg Cys Ile
1               5                   10


<210>  61
<211>  60
<212>  PRT
<213>  Artificial Sequence

<220>
<223>  Cardiotoxin

<400>  61

Leu Lys Cys Asn Lys Leu Val Pro Leu Phe Tyr Lys Thr Cys Pro Ala
1               5                   10                  15


Gly Lys Asn Leu Cys Tyr Lys Met Phe Met Val Ser Asn Lys Met Val
            20                  25                  30


Pro Val Lys Arg Gly Cys Ile Asp Val Cys Pro Lys Ser Ser Leu Leu
        35                  40                  45


Val Lys Tyr Val Cys Cys Asn Thr Asp Arg Cys Asn
    50                  55                  60


<210>  62
<211>  58
<212>  PRT
<213>  Artificial Sequence

<220>
<223>  BL-8040

<400>  62

Arg Pro Asp Phe Cys Leu Glu Pro Pro Tyr Thr Gly Pro Cys Arg Ala
1               5                   10                  15


Arg Ile Ile Arg Tyr Phe Tyr Asn Ala Lys Ala Gly Leu Cys Gln Thr
            20                  25                  30


Phe Val Val Gly Gly Cys Arg Ala Lys Arg Asn Asn Phe Lys Ser Ala
        35                  40                  45


Glu Asp Cys Glu Arg Thr Cys Gly Gly Ala
    50                  55


<210>  63

<211>  14
<212>  PRT
<213>  Artificial Sequence

<220>
<223>  BL-8040


<220>
<221>  MISC_FEATURE
<222>  (3)..(3)
<223>  Xaa is Nal

<220>
<221>  MISC_FEATURE
<222>  (6)..(6)
<223>  Xaa is Cit

<220>
<221>  MISC_FEATURE
<222>  (12)..(12)
<223>  Xaa is Cit

<400>  63

Arg Arg Xaa Cys Tyr Xaa Lys Lys Pro Tyr Arg Xaa Cys Arg
1               5                   10


<210>  64
<211>  9
<212>  PRT
<213>  Artificial Sequence

<220>
<223>  CBLB-612

<400>  64

Val Gln Gly Glu Glu Ser Asn Asp Lys
1               5


<210>  65
<211>  21
<212>  PRT
<213>  Artificial Sequence

<220>
<223>  EPO-018B

<400>  65

Gly Gly Thr Tyr Ser Cys His Phe Gly Ala Leu Thr Trp Val Cys Arg
1               5                   10                  15


Pro Gln Arg Gly Ala
            20


<210>  66
<211>  7

<212>  PRT
<213>  Artificial Sequence

<220>
<223>  Etelcalcetide Hydrochloride

<400>  66

Cys Ala Arg Arg Arg Ala Arg
1               5


<210>  67
<211>  301
<212>  PRT
<213>  Artificial Sequence

<220>
<223>  MOD-4023

<400>  67

Met Ala Thr Gly Ser Arg Thr Ser Leu Leu Leu Ala Phe Gly Leu Leu
1               5                   10                  15

Cys Leu Pro Trp Leu Gln Glu Gly Ser Ala Ser Ser Ser Ser Lys Ala
            20                  25                  30

Pro Pro Pro Ser Leu Pro Ser Pro Ser Arg Leu Pro Gly Pro Ser Asp
        35                  40                  45

Thr Pro Ile Leu Pro Gln Phe Pro Thr Ile Pro Leu Ser Arg Leu Phe
    50                  55                  60

Asp Asn Ala Met Leu Arg Ala His Arg Leu His Gln Leu Ala Phe Asp
65                  70                  75                  80

Thr Tyr Gln Glu Phe Glu Glu Ala Tyr Ile Pro Lys Glu Gln Lys Tyr
                85                  90                  95

Ser Phe Leu Gln Asn Pro Gln Thr Ser Leu Cys Phe Ser Glu Ser Ile
            100                 105                 110

Pro Thr Pro Ser Asn Arg Glu Glu Thr Gln Gln Lys Ser Asn Leu Glu
        115                 120                 125

Leu Leu Arg Ile Ser Leu Leu Leu Ile Gln Ser Trp Leu Glu Pro Val
    130                 135                 140

Gln Phe Leu Arg Ser Val Phe Ala Asn Ser Leu Val Tyr Gly Ala Ser
145                 150                 155                 160

Asp Ser Asn Val Tyr Asp Leu Leu Lys Asp Leu Glu Glu Gly Ile Gln

165                 170                 175

Thr Leu Met Gly Arg Leu Glu Asp Gly Ser Pro Arg Thr Gly Gln Ile
            180                 185                 190

Phe Lys Gln Thr Tyr Ser Lys Phe Asp Thr Asn Ser His Asn Asp Asp
        195                 200                 205

Ala Leu Leu Lys Asn Tyr Gly Leu Leu Tyr Cys Phe Arg Lys Asp Met
    210                 215                 220

Asp Lys Val Glu Thr Phe Leu Arg Ile Val Gln Cys Arg Ser Val Glu
225                 230                 235                 240

Gly Ser Cys Gly Phe Ser Ser Ser Ser Lys Ala Pro Pro Pro Ser Leu
                245                 250                 255

Pro Ser Pro Ser Arg Leu Pro Gly Pro Ser Asp Thr Pro Ile Leu Pro
            260                 265                 270

Gln Ser Ser Ser Ser Lys Ala Pro Pro Pro Ser Leu Pro Ser Pro Ser
        275                 280                 285

Arg Leu Pro Gly Pro Ser Asp Thr Pro Ile Leu Pro Gln
    290                 295                 300

<210>  68
<211>  16
<212>  PRT
<213>  Artificial Sequence

<220>
<223>  ATI-2341

<400>  68

Met Gly Tyr Gln Lys Lys Leu Arg Ser Met Thr Asp Lys Tyr Arg Leu
1               5                   10                  15

<210>  69
<211>  19
<212>  PRT
<213>  Artificial Sequence

<220>
<223>  Edratide

<400>  69

Gly Tyr Tyr Trp Ser Trp Ile Arg Gln Pro Pro Gly Lys Gly Glu Glu
1               5                   10                  15

Trp Ile Gly

<210> 70
<211> 21
<212> PRT
<213> Artificial Sequence

<220>
<223> Forigerimod Acetate

<220>
<221> MISC_FEATURE
<222> (10)..(10)
<223> Xaa is DL-threonine

<400> 70

Arg Ile His Met Val Tyr Ser Lys Arg Xaa Gly Lys Pro Arg Gly Tyr
1               5                   10                  15

Ala Phe Ile Glu Tyr
            20

<210> 71
<211> 37
<212> PRT
<213> Artificial Sequence

<220>
<223> hPTH-1-37

<400> 71

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe Val Ala Leu
        35

<210> 72
<211> 16
<212> PRT
<213> Artificial Sequence

<220>
<223> ALB-408

<400> 72

Leu Val Arg Tyr Thr Lys Lys Val Pro Gln Val Ser Thr Pro Thr Leu
1               5                   10                  15

<210>  73
<211>  36
<212>  PRT
<213>  Artificial Sequence

<220>
<223>  Enfuvirtide

<400>  73

Tyr Thr Ser Leu Ile His Ser Leu Ile Glu Glu Ser Gln Asn Gln Gln
1               5                   10                  15

Glu Lys Asn Glu Gln Glu Leu Leu Glu Leu Asp Lys Trp Ala Ser Leu
            20                  25                  30

Trp Asn Trp Phe
        35

<210>  74
<211>  34
<212>  PRT
<213>  Artificial Sequence

<220>
<223>  FB-006M

<400>  74

Trp Glu Glu Trp Asp Arg Glu Ile Asn Asn Tyr Thr Lys Leu Ile His
1               5                   10                  15

Glu Leu Ile Glu Glu Ser Gln Asn Gln Gln Glu Lys Asn Glu Gln Glu
            20                  25                  30

Leu Leu

<210>  75
<211>  28
<212>  PRT
<213>  Artificial Sequence

<220>
<223>  Thymalfasin

<400>  75

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn
            20                  25

<210>  76
<211>  36
<212>  PRT
<213>  Artificial Sequence

<220>
<223>  Sifuvirtide

<400>  76

Ser Trp Glu Thr Trp Glu Arg Glu Ile Glu Asn Tyr Thr Arg Gln Ile
1               5                   10                  15

Tyr Arg Ile Leu Glu Glu Ser Gln Glu Gln Gln Asp Arg Asn Glu Arg
            20                  25                  30

Asp Leu Leu Glu
        35

<210>  77
<211>  28
<212>  PRT
<213>  Artificial Sequence

<220>
<223>  Thymalfasin

<400>  77

Ser Asp Ala Ala Val Asp Thr Ser Ser Glu Ile Thr Thr Lys Asp Leu
1               5                   10                  15

Lys Glu Lys Lys Glu Val Val Glu Glu Ala Glu Asn
            20                  25

<210>  78
<211>  40
<212>  PRT
<213>  Artificial Sequence

<220>
<223>  VIR-576

<400>  78

Leu Glu Ala Ile Pro Cys Ser Ile Pro Pro Glu Phe Leu Phe Gly Lys
1               5                   10                  15

Pro Phe Val Phe Leu Glu Ala Ile Pro Cys Ser Ile Pro Pro Glu Phe
            20                  25                  30

Leu Phe Gly Lys Pro Phe Val Phe
        35                  40

<210>  79

<211>  37
<212>  PRT
<213>  Artificial Sequence

<220>
<223>  AC-163794

<400>  79

Glu Gly Thr Phe Ile Ser Asp Tyr Ser Ile Ala Met Asp Lys Ile His
1               5                   10                  15

Gln Gln Asp Phe Val Asn Trp Leu Leu Ala Gln Lys Pro Ser Ser Gly
            20                  25                  30

Ala Pro Pro Pro Ser
        35

<210>  80
<211>  32
<212>  PRT
<213>  Artificial Sequence

<220>
<223>  Calcitonin

<400>  80

Cys Gly Asn Leu Ser Thr Cys Met Leu Gly Thr Tyr Thr Gln Asp Phe
1               5                   10                  15

Asn Lys Phe His Thr Phe Pro Gln Thr Ala Ile Gly Val Gly Ala Pro
            20                  25                  30

<210>  81
<211>  7
<212>  PRT
<213>  Artificial Sequence

<220>
<223>  DD-04107

<220>
<221>  MISC_FEATURE
<222>  (1)..(1)
<223>  Xaa is a palmitoyl-

<400>  81

Xaa Glu Glu Met Gln Arg Arg
1               5

<210>  82
<211>  31
<212>  PRT
<213>  Artificial Sequence

<220>
<223>  Exendin-(9-39)

<400>  82

Asp Leu Ser Lys Gln Met Glu Glu Glu Ala Val Arg Leu Phe Ile Glu
1               5                   10                  15

Trp Leu Lys Asn Gly Gly Pro Ser Ser Gly Ala Pro Pro Pro Ser
            20                  25                  30

<210>  83
<211>  14
<212>  PRT
<213>  Artificial Sequence

<220>
<223>  HIP-2B

<400>  83

Ile Gly Leu His Asp Pro Thr Gln Gly Thr Glu Pro Asn Gly
1               5                   10

<210>  84
<211>  37
<212>  PRT
<213>  Artificial Sequence

<220>
<223>  HS-20004

<400>  84

Glu Gly Thr Phe Thr Ser Asp Val Ser Ser Tyr Leu Glu Glu Glu Ala
1               5                   10                  15

Ala Lys Glu Phe Ile Ala Trp Leu Val Arg Gly Gly Pro Ser Ser Gly
            20                  25                  30

Ala Pro Pro Pro Ser
        35

<210>  85
<211>  51
<212>  PRT
<213>  Artificial Sequence

<220>
<223>  insulin aspart

<220>
<221>  DISULFID
<222>  (7)..(37)

<220>
<221>  DISULFID
<222>  (19)..(50)

<220>
<221>  DISULFID
<222>  (36)..(41)

<400>  85

Phe Val Asn Gln His Leu Cys Gly Ser His Leu Val Glu Ala Leu Tyr
1               5                   10                  15

Leu Val Cys Gly Glu Arg Gly Phe Phe Tyr Thr Asp Lys Thr Gly Ile
            20                  25                  30

Val Glu Gln Cys Cys Thr Ser Ile Cys Ser Leu Tyr Gln Leu Glu Asn
        35                  40                  45

Tyr Cys Asn
    50

<210>  86
<211>  50
<212>  PRT
<213>  Artificial Sequence

<220>
<223>  insulin degludec

<220>
<221>  DISULFID
<222>  (7)..(36)

<220>
<221>  DISULFID
<222>  (19)..(49)

<220>
<221>  DISULFID
<222>  (35)..(40)

<400>  86

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Gly Ile Val
            20                  25                  30

Glu Gln Cys Cys Thr Ser Ile Cys Ser Leu Tyr Gln Leu Glu Asn Tyr
        35                  40                  45

Cys Asn
    50

<210>  87
<211>  50
<212>  PRT
<213>  Artificial Sequence

<220>
<223>  insulin detemir


<220>
<221>  DISULFID
<222>  (7)..(36)

<220>
<221>  DISULFID
<222>  (19)..(49)

<220>
<221>  DISULFID
<222>  (35)..(40)

<400>  87

Phe Val Asn Gln His Leu Cys Gly Ser His Leu Val Glu Ala Leu Tyr
1               5                   10                  15


Leu Val Cys Gly Glu Arg Gly Phe Phe Tyr Thr Pro Lys Gly Ile Val
            20                  25                  30


Glu Gln Cys Cys Thr Ser Ile Cys Ser Leu Tyr Gln Leu Glu Asn Tyr
        35                  40                  45


Cys Asn
    50


<210>  88
<211>  53
<212>  PRT
<213>  Artificial Sequence

<220>
<223>  insulin glargine


<220>
<221>  DISULFID
<222>  (7)..(39)

<220>
<221>  DISULFID
<222>  (19)..(52)

<220>
<221>  DISULFID
<222>  (38)..(43)

<400>  88

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Gly Ile Val Glu Gln Cys Cys Thr Ser Ile Cys Ser Leu Tyr Gln Leu
        35                  40                  45

Glu Asn Tyr Cys Gly
    50

<210>  89
<211>  51
<212>  PRT
<213>  Artificial Sequence

<220>
<223>  insulin glulisine

<220>
<221>  DISULFID
<222>  (7)..(37)

<220>
<221>  DISULFID
<222>  (19)..(50)

<220>
<221>  DISULFID
<222>  (36)..(41)

<400>  89

Phe Val Lys Gln His Leu Cys Gly Ser His Leu Val Glu Ala Leu Tyr
1               5                   10                  15

Leu Val Cys Gly Glu Arg Gly Phe Phe Tyr Thr Pro Glu Thr Gly Ile
            20                  25                  30

Val Glu Gln Cys Cys Thr Ser Ile Cys Ser Leu Tyr Gln Leu Glu Asn
        35                  40                  45

Tyr Cys Asn
    50

<210>  90
<211>  51
<212>  PRT
<213>  Artificial Sequence

<220>
<223>  insulin lispro

<220>
<221> DISULFID
<222> (7)..(37)

<220>
<221> DISULFID
<222> (19)..(50)

<220>
<221> DISULFID
<222> (36)..(41)

<400> 90

Phe Val Asn Gln His Leu Cys Gly Ser His Leu Val Glu Ala Leu Tyr
1               5                   10                  15

Leu Val Cys Gly Glu Arg Gly Phe Phe Tyr Thr Lys Pro Thr Gly Ile
            20                  25                  30

Val Glu Gln Cys Cys Thr Ser Ile Cys Ser Leu Tyr Gln Leu Glu Asn
        35                  40                  45

Tyr Cys Asn
    50

<210> 91
<211> 51
<212> PRT
<213> Artificial Sequence

<220>
<223> insulin neutral

<220>
<221> DISULFID
<222> (7)..(37)

<220>
<221> DISULFID
<222> (19)..(50)

<220>
<221> DISULFID
<222> (36)..(41)

<400> 91

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Ala Gly Ile
            20                  25                  30

Val Glu Gln Cys Cys Thr Ser Ile Cys Ser Leu Tyr Gln Leu Glu Asn
        35                  40                  45

Tyr Cys Asn
    50

<210>  92
<211>  44
<212>  PRT
<213>  Artificial Sequence

<220>
<223>  Lixisenatide

<400>  92

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Ser Lys Lys Lys Lys Lys Lys
        35                  40

<210>  93
<211>  39
<212>  PRT
<213>  Artificial Sequence

<220>
<223>  PEX-168

<400>  93

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  94
<211>  22
<212>  PRT
<213>  Artificial Sequence

<220>
<223>  Pexiganan

<400>  94

Gly Ile Gly Lys Phe Leu Lys Lys Ala Lys Lys Phe Gly Lys Ala Phe

1               5                   10                  15

Val Lys Leu Leu Lys Lys
            20

<210>  95
<211>  5
<212>  PRT
<213>  Artificial Sequence

<220>
<223>  PMZ-2123

<400>  95

Asp Pro Val Leu Trp
1               5

<210>  96
<211>  34
<212>  PRT
<213>  Artificial Sequence

<220>
<223>  Teriparatide

<400>  96

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

<210>  97
<211>  34
<212>  PRT
<213>  Artificial Sequence

<220>
<223>  teriparatide acetate

<400>  97

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe

<210>  98
<211>  44
<212>  PRT
<213>  Artificial Sequence

<220>
<223>  tesamorelin acetate

<220>
<221>  MISC_FEATURE
<222>  (1)..(1)
<223>  Xaa is N-[(3E)-1-oxo-3-hexenyl]-

<400>  98

Xaa Tyr Ala Asp Ala Ile Phe Thr Asn Ser Tyr Arg Lys Leu Gly Gln
1               5                   10                  15

Leu Ser Ala Arg Lys Leu Leu Gln Asp Ile Met Ser Arg Gln Gln Gly
            20                  25                  30

Glu Ser Asn Gln Glu Arg Gly Ala Arg Ala Arg Leu
        35                  40

<210>  99
<211>  5
<212>  PRT
<213>  Artificial Sequence

<220>
<223>  VTC-G15

<400>  99

Leu Val Lys Gly Arg
1               5

<210>  100
<211>  29
<212>  PRT
<213>  Artificial Sequence

<220>
<223>  ZP-4207

<400>  100

His Ser Gln Gly Thr Phe Thr Ser Asp Tyr Ser Lys Tyr Leu Asp Arg
1               5                   10                  15

Ala Arg Ala Asp Asp Phe Val Ala Trp Leu Lys Ser Thr
            20                  25

<210>  101

<211>  24
<212>  PRT
<213>  Artificial Sequence

<220>
<223>  AD-01

<400>  101

Gln Ile Arg Gln Gln Pro Arg Asp Pro Pro Thr Glu Thr Leu Glu Leu
1               5                   10                  15

Glu Val Ser Pro Asp Pro Ala Ser
            20

<210>  102
<211>  25
<212>  PRT
<213>  Artificial Sequence

<220>
<223>  ATSP-7041

<400>  102

Cys Lys Gly Gly Arg Ala Lys Asp Cys Gly Gly Lys Leu Ala Lys Leu
1               5                   10                  15

Ala Lys Lys Leu Ala Lys Leu Ala Lys
            20                  25

<210>  103
<211>  12
<212>  PRT
<213>  Artificial Sequence

<220>
<223>  BMTP-11

<220>
<221>  MISC_FEATURE
<222>  (9)..(9)
<223>  Xaa is Cba

<400>  103

Leu Thr Phe Glu Tyr Trp Ala Gln Xaa Ser Ala Ala
1               5                   10

<210>  104
<211>  26
<212>  PRT
<213>  Artificial Sequence

<220>
<223>  BMTP-11

<400>  104

Cys Gly Arg Arg Ala Gly Gly Ser Cys Gly Gly Asp Lys Leu Ala Lys
1               5                   10                  15

Leu Ala Lys Lys Leu Ala Lys Leu Ala Lys
            20                  25

<210>  105
<211>  6
<212>  PRT
<213>  Artificial Sequence

<220>
<223>  Box-5

<400>  105

Met Asp Gly Cys Glu Leu
1               5

<210>  106
<211>  16
<212>  PRT
<213>  Artificial Sequence

<220>
<223>  Dolcanatide

<400>  106

Asn Asp Glu Cys Glu Leu Cys Val Asn Val Ala Cys Thr Gly Cys Leu
1               5                   10                  15

<210>  107
<211>  5
<212>  PRT
<213>  Artificial Sequence

<220>
<223>  Dusquetide

<400>  107

Arg Ile Val Pro Ala
1               5

<210>  108
<211>  6
<212>  PRT
<213>  Artificial Sequence

<220>
<223>  Foxy-5

<400>  108

Met Asp Gly Cys Glu Leu

1                   5


<210>  109
<211>  16
<212>  PRT
<213>  Artificial Sequence

<220>
<223>  GO-2032c

<400>  109

Arg Arg Arg Arg Arg Arg Arg Arg Arg Cys Gln Cys Arg Arg Lys Asn
1               5                   10                  15


<210>  110
<211>  9
<212>  PRT
<213>  Artificial Sequence

<220>
<223>  LTX-315



<220>
<221>  MISC_FEATURE
<222>  (8)..(8)
<223>  Xaa id Dip

<400>  110

Lys Lys Trp Trp Lys Lys Trp Xaa Lys
1               5


<210>  111
<211>  8
<212>  PRT
<213>  Artificial Sequence

<220>
<223>  LY-2510924



<220>
<221>  MISC_FEATURE
<222>  (5)..(5)
<223>  Xaa is Nal

<400>  111

Phe Tyr Lys Arg Xaa Gly Glu Lys
1               5


<210>  112
<211>  14
<212>  PRT
<213>  Artificial Sequence

<220>

<223>  SORC-13

<400>  112

Lys Ile Glu Phe Leu His Pro Ser Lys Val Asp Leu Pro Arg
1               5                   10

<210>  113
<211>  14
<212>  PRT
<213>  Artificial Sequence

<220>
<223>  B27-PD

<400>  113

Ala Leu Asn Glu Asp Leu Ser Ser Trp Thr Ala Ala Asp Thr
1               5                   10

<210>  114
<211>  12
<212>  PRT
<213>  Artificial Sequence

<220>
<223>  C-16Y

<400>  114

Asp Phe Lys Leu Phe Ala Val Tyr Ile Lys Tyr Arg
1               5                   10

<210>  115
<211>  44
<212>  PRT
<213>  Artificial Sequence

<220>
<223>  RGN-259

<400>  115

Met Ser Asp Lys Pro Asp Met Ala Glu Ile Glu Lys Phe Asp Lys Ser
1               5                   10                  15

Lys Leu Lys Lys Thr Glu Thr Gln Glu Lys Asn Pro Leu Pro Ser Lys
            20                  25                  30

Glu Thr Ile Glu Gln Glu Lys Gln Ala Gly Glu Ser
        35                  40

<210>  116
<211>  32
<212>  PRT
<213>  Artificial Sequence

<220>
<223>  CGEN-25009

<400>  116

Gly Gln Lys Gly Gln Val Gly Pro Pro Gly Ala Ala Val Arg Arg Ala
1               5                   10                  15

Tyr Ala Ala Phe Ser Val Gly Arg Arg Ala Tyr Ala Ala Phe Ser Val
            20                  25                  30

<210>  117
<211>  21
<212>  PRT
<213>  Artificial Sequence

<220>
<223>  Lucinactant

<400>  117

Lys Leu Leu Leu Leu Lys Leu Leu Leu Leu Lys Leu Leu Leu Leu Lys
1               5                   10                  15

Leu Leu Leu Leu Lys
            20

<210>  118
<211>  22
<212>  PRT
<213>  Artificial Sequence

<220>
<223>  MMI-0100

<400>  118

Tyr Ala Arg Ala Ala Ala Arg Gln Ala Arg Ala Lys Ala Leu Ala Arg
1               5                   10                  15

Gln Leu Gly Val Ala Ala
            20

<210>  119
<211>  10
<212>  PRT
<213>  Artificial Sequence

<220>
<223>  Gonadorelin

<220>
<221>  MISC_FEATURE
<222>  (1)..(1)
<223>  Xaa is pyroglutamic acid

<400>  119

Xaa His Trp Ser Tyr Gly Leu Arg Pro Gly
1               5                   10


<210>  120
<211>  10
<212>  PRT
<213>  Artificial Sequence

<220>
<223>  Nafarelin


<220>
<221>  MISC_FEATURE
<222>  (1)..(1)
<223>  Xaa is pyroglutamic acid

<220>
<221>  MISC_FEATURE
<222>  (6)..(6)
<223>  Xaa is NaI

<400>  120

Xaa His Trp Ser Tyr Xaa Leu Arg Pro Gly
1               5                   10


<210>  121
<211>  9
<212>  PRT
<213>  Artificial Sequence

<220>
<223>  Oxytocin

<400>  121

Cys Tyr Ile Gln Asn Cys Pro Leu Gly
1               5


<210>  122
<211>  9
<212>  PRT
<213>  Artificial Sequence

<220>
<223>  Urofollitropin

<400>  122

Cys Tyr Ile Thr Asn Cys Pro Leu Gly
1               5